# EUROPÄISCHE PATENTSCHRIFT

(11) **EP 0 595 130 B1**
(45) Veröffentlichungstag und Bekanntmachung des Hinweises auf die Patenterteilung: **03.07.1996**
(21) Anmeldenummer: 93116688.8
(22) Anmeldetag: 15.10.1993
(51) Int. Cl.: C07C 255/29, C07C 255/31, C07C 255/46, C07C 323/59, C07D 317/72, C07D 333/38, C07D 211/66, C07D 335/02, C07C 233/52, C07C 233/51

(54) **N-Phenylacetaminonitrile und ihre Verwendung als Zwischenprodukte zur Synthese von Insektiziden und Herbiziden 3-Aryl-pyrrolidin-2,4-dionen**
N-phenylacetaminonitriles and their use as intermediates for the synthesis of insecticidal and herbicidal 3-aryl-pyrrolidine-2,4-diones
N-phénylacétaminonitriles et leur utilisation comme intermédiaires dans la synthèse de 3-aryl-pyrrolidine-2,4-diones ayant une activité herbicide et insecticide

(30) Priorität: 28.10.1992 DE 4236400
(43) Veröffentlichungstag der Anmeldung: 04.05.1994
(73) Patentinhaber: BAYER AG, 51368 Leverkusen (DE)
(72) Erfinder: Fischer, Reiner, Dr., D-40789 Monheim (DE); Beck, Gunther, Dr., D-51375 Leverkusen (DE)

(56) Entgegenhaltungen:
- EP-A- 0 427 907
- EP-A- 0 456 063
- GB-A- 419 457
- LIEBIGS ANNALEN DER CHEMIE Bd. 764 , 1972 , WEINHEIM DE Seiten 69 - 93 P. KURTZ, H. DISSELNKÖTTER 'Enamide'
- JOURNAL OF ORGANIC CHEMISTRY Bd. 43 , 1978 , EASTON US Seiten 2576 - 2581 A. ROMEO ET AL. 'Approach to the use of Benzylpenicillinacylase for Configurational Correlation of amino Compounds'
- CHEMICAL ABSTRACTS, vol. 68, no. 3, 15. Januar 1968, Columbus, Ohio, US; abstract no. 12942a, V. G. GRANIK, R. G. GLUSHKOV 'Lactams. X. The synthesis of 9H-pyrimido[4,5-b]-azepine derivatives from caprolactam.' Seite 1243 ;
- 'Organic Synthesis vol. 22' 1942 , JOHN WILEY & SONS , NEW YORK (US)

## Beschreibung

Die Erfindung betrifft neue N-Phenylacetaminonitrile, ein Verfahren zu ihrer Herstellung und ihre Verwendung als Zwischenprodukte zur Synthese von insektiziden, akariziden und herbiziden 3-Aryl-pyrrolidin-2,4-dionen.

Die Herstellung von 3-Aryl-pyrrolidin-2,4-dionen aus N-Phenylacetaminocarbonsäureestern ist bekannt (vergl. z.B. EP 456 063). Die hierfür als Vorprodukte erforderlichen N-Phenylacetaminocarbonsäureester werden in der Regel mit Hilfe einer vierstufigen Reaktionsfolge erhalten, indem man zunächst die entsprechenden Aminosäuren in zwei Stufen über Keton- und Aminonitrilzwischenstufen herstellt und isoliert und dann entweder zunächst acyliert und dann verestert oder zuerst verestert und anschließend acyliert (vergl. z.B. Indiau J. Chem. 6, 341-345 [1968]; EP 456 063).

Die Herstellung von N-Phenylacetaminonitrilen durch Umsetzung von Aminonitrilen mit Phenylessigsäurechloriden in Gegenwart von Basen ist ebenfalls bekannt (vergl. z.B. J. Org. Chem. 43, 2576-2581 [1978]).

Es wurden neue N-Phenylacetaminonitrile der allgemeinen Formel (I), 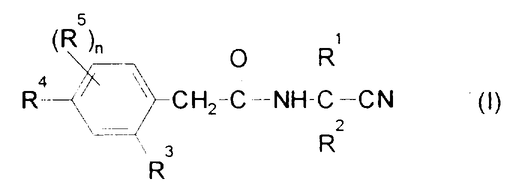 in welcher
- R¹: für Wasserstoff oder für jeweils gegebenenfalls substituiertes Alkyl, Alkenyl, Cycloalkyl, Aryl oder Heterocyclyl steht und
- R²: für Wasserstoff oder für gegebenenfalls substituiertes Alkyl steht oder
- R¹ und R²: gemeinsam mit dem Kohlenstoffatom, an welches sie gebunden sind, für jeweils gegebenenfalls substituiertes Cycloalkyl oder Heterocyclyl stehen,
- R³: für Halogen, Alkyl oder Alkoxy steht,
- R⁴: für Wasserstoff, Halogen, Alkyl, Halogenalkyl oder Alkoxy steht,
- R⁵: für Halogen, Alkyl oder Alkoxy steht und
- n: für eine Zahl 0, 1, 2 oder 3 steht,
gefunden.

Die Verbindungen der Formel (I) können gegebenenfalls in Abhängigkeit von der Art der Substituenten als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefinden, daß man die neuen N-Phenylacetaminonitrile der allgemeinen Formel (I), 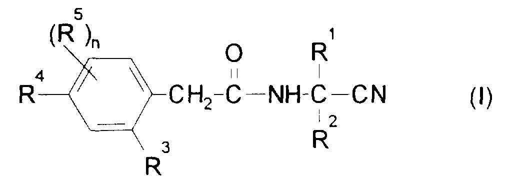 in welcher
- R¹: für Wasserstoff oder für jeweils gegebenenfalls substituiertes Alkyl, Alkenyl, Cycloalkyl, Aryl oder Heterocyclyl steht und
- R²: für Wasserstoff oder für gegebenenfalls substituiertes Alkyl steht oder
- R¹ und R²: gemeinsam mit dem Kohlenstoffatom, an welches sie gebunden sind, für jeweils gegebenenfalls substituiertes Cycloalkyl oder Heterocyclyl stehen,
- R³: für Halogen, Alkyl oder Alkoxy steht,
- R⁴: für Wasserstoff, Halogen, Alkyl, Halogenalkyl oder Alkoxy steht,
- R⁵: für Halogen, Alkyl oder Alkoxy steht und
- n: für eine Zahl 0, 1, 2 oder 3 steht,
erhält, wenn man α-Aminonitrile der Formel (II), 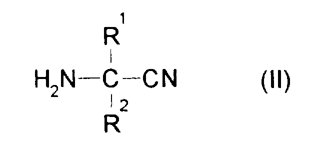 in welcher
R¹ und R² die oben angegebene Bedeutung haben,
mit Phenylessigsäurehalogeniden der Formel (III), 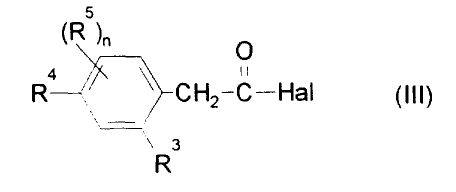 in welcher
R³, R⁴, R⁵ und ndie oben angegebene Bedeutung haben und
Hal für Halogen steht,
gegebenenfalls in Gegenwart eines Verdünnungsmittels und gegebenenfalls in Gegenwart eines Reaktionshilfsmittels umsetzt.

Schließlich wurde gefunden, daß die neuen N-Phenylacetaminonitrile der Formel (1) sich hervorragend als leicht zugängliche Zwischenprodukte zur Synthese von insektiziden, akariziden und herbiziden 3-Aryl-pyrrolidin-2,4-dionen eignen, indem man sie zunächst in einer ersten Stufe mit Alkoholen der Formel (IV),

R-OH (V)

in welcher
R für Alkyl steht,
gegebenenfalls in Gegenwart eines Verdünnungsmittels und in Gegenwart von Schwefelsäure als Reaktionshilfsmittels umsetzt und dann die so erhältlichen N-Phenylacetaminocarbonsäureester der Formel (V), 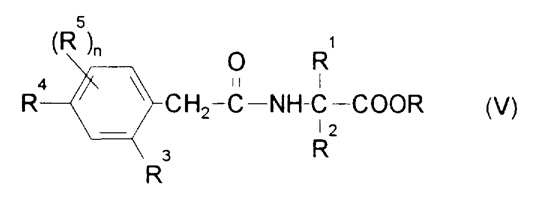 in welcher
R, R¹, R², R³, R⁴, R⁵ und n die oben angegebene Bedeutung haben,
in einer anschließenden 2.Stufe gegebenenfalls in Gegenwart eines Verdünnungsmittels und gegebenenfalls in Gegenwart eines Reaktionshilfsmittels cyclisiert.

Dabei war es besonders überraschend, daß die Umsetzung der N-Phenylacetaminonitrile der Formel (I) mit Alkoholen der Formel (IV) in Gegenwart von Schwefelsäure als Reaktionshilfsmittel in hoher Ausbeute und Reinheit die gewünschten N-Phenylacetaminocarbonsäureester der Formel (V) lieferte, da eine entsprechende Umsetzung analog zu literaturbekannten Verfahren (vergl. z.B. Khim. Farm. Zh. 1, 21-26 [1967] bzw. CA 68: 12942a) mit Salzsäure als Reaktionshilfsmittel nicht die gewünschten Produkte lieferte.

Als besonderer Vorteil dieser Vorgehensweise erweist es sich, daß in der Synthese der als Endprodukte erwünschten 3-Aryl-pyrrolidin-2,4-dione die Aminosäurezwischenstufe, die wegen ihres zwitterionischen Charakters in der Isolierung und Reinigung besondere Probleme bereitet, nicht mehr auftritt und daß sich außerdem die Gesamtsynthese von vier auf drei Stufen reduziert.

Die erfindungsgemäßen N-Phenylacetaminonitrile sind durch die Formel (I) allgemein definiert. Bevorzugt sind Verbindungen der Formel (I), bei welchen
- R¹: für Wasserstoff, für jeweils geradkettiges oder verzweigtes Alkyl mit 1 bis 12 Kohlenstoffatomen, Alkenyl mit 3 bis 8 Kohlenstoffatomen, Halogenalkyl mit 1 bis 8 Kohlenstoffatomen und 1 bis 17 gleichen oder verschiedenen Halogenatomen, Alkoxyalkyl mit jeweils 1 bis 8 Kohlenstoffatomen in den einzelnen Alkylteilen, Alkoxyalkoxyalkyl mit jeweils 1 bis 8 Kohlenstoffatomen in den einzelnen Alkylteilen, Alkylthioalkyl mit jeweils 1 bis 8 Kohlenstoffatomen in den einzelnen Alkylteilen, Cycloalkyl mit 3 bis 8 Kohlenstoffatomen, gesättigtes Heterocyclyl mit 3 bis 8 Ringgliedern und 1 bis 2 gleichen oder verschiedenen Heteroatomen - insbesondere Stickstoff, Sauerstoff und/oder Schwefel - oder für jeweils gegebenenfalls im Aryl- bzw. Heteroarylteil einfach oder mehrfach, gleich oder verschieden substituiertes Arylalkyl oder Aryl mit jeweils 6 bis 10 Kohlenstoffatomen im Arylteil und gegebenenfalls 1 bis 6 Kohlenstoffatomen im geradkettigen oder verzweigten Alkylteil oder Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Aryl- bzw. Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen sowie jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen und
- R²: für Wasserstoff, für jeweils geradkettiges oder verzweigtes Alkyl mit 1 bis 12 Kohlenstoffatomen, Halogenalkyl mit 1 bis 8 Kohlenstoffatomen und 1 bis 17 gleichen oder verschiedenen Halogenatomen oder Alkoxyalkyl mit jeweils 1 bis 8 Kohlenstoffatomen in den einzelnen Alkylteilen steht oder
- R¹ und R²: gemeinsam mit dem Kohlenstoffatom, an welches sie gebunden sind, für jeweils gegebenenfalls einfach oder mehrfach, gleich oder verschieden substituiertes, gesättigtes oder ungesättigtes Cycloalkyl oder Heterocyclyl mit jeweils 3 bis 12 Kohlenstoffatomen und gegebenenfalls 1 bis 3 gleichen oder verschiedenen Heteroatomen - insbesondere Stickstoff, Sauerstoff und/oder Schwefel - stehen, wobei als Substituenten jeweils infrage kommen:
Halogen, geradkettiges oder verzweigtes Alkyl mit 1 bis 6 Kohlenstoffatomen, geradkettiges oder verzweigtes Alkanoyl mit 1 bis 7 Kohlenstoffatomen, geradkettiges oder verzweigtes Alkandiyl mit 3 bis 8 Kohlenstoffatomen, geradkettiges oder verzweigtes Dioxyalkylen mit 1 bis 8 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxy mit 1 bis 6 Kohlenstoffatomen, geradkettiges oder verzweigtes Alkylthio mit 1 bis 6 Kohlenstoffatomen, Cycloalkyl mit 3 bis 8 Kohlenstoffatomen, Aryl mit 6 bis 10 Kohlenstoffatomen,
- R³: für Fluor, Chlor, Brom, Iod, für geradkettiges oder verzweigtes Alkyl mit 1 bis 8 Kohlenstoffatomen oder für geradkettiges oder verzweigtes Alkoxy mit 1 bis 8 Kohlenstoffatomen steht,
- R⁴: für Wasserstoff für Fluor, Chlor, Brom, lod, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für geradkettiges oder verzweigtes Alkoxy mit 1 bis 8 Kohlenstoffatomen steht,
- R⁵: für Fluor, Chlor, Brom, lod, für geradkettiges oder verzweigtes Alkyl mit 1 bis 8 Kohlenstoffatomen oder für geradkettiges oder verzweigtes Alkoxy mit 1 bis 8 Kohlenstoffatomen steht und
- n: für eine Zahl 0, 1, 2 oder 3 steht.

Besonders bevorzugt sind Verbindungen der Formel (I), bei welchen
- R¹: für Wasserstoff, für jeweils geradkettiges oder verzweigtes Alkyl mit 1 bis 10 Kohlenstoffatomen, Alkenyl mit 3 bis 6 Kohlenstoffatomen, Halogenalkyl mit 1 bis 6 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, Alkoxyalkoxyalkyl mit jeweils 1 bis 6 Kohlenstoffatomen in den einzelnen Alkylteilen, Alkylthioalkyl mit jeweils 1 bis 6 Kohlenstoffatomen in den einzelnen Alkylteilen, Cycloalkyl mit 3 bis 7 Kohlenstoffatomen, gesättigtes Heterocyclyl mit 3 bis 7 Ringgliedern und 1 oder 2 gleichen oder verschiedenen Heteroatomen - insbesondere Stickstoff Sauerstoff und/oder Schwefel - oder für jeweils gegebenenfalls im Aryl- bzw. Heteroarylteil einfach bis fünffach, gleich oder verschieden substituiertes Arylalkyl oder Aryl mit jeweils 6 oder 10 Kohlenstoffatomen im Arylteil und gegebenenfalls 1 bis 4 Kohlenstoffatomen im geradkettigen oder verzweigten Alkylteil oder Heteroaryl mit 2 bis 9 Kohlenstoffatomen und 1 bis 3 gleichen oder verschiedenen Heteroatomen - insbesondere Stickstoff, Sauerstoffund/oder Schwefel - steht, wobei als Aryl- bzw. Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen sowie jeweils geradkettiges oder verzweigtes Halogenalkyl, Halogenalkoxy, Halogenalkylthio. Halogenalkylsulfinyl oder Halogenalkylsulfonyl mit jeweils 1 bis 4 Kohlenstoffatomen und 1 bis 9 gleichen oder verschiedenen Halogenatomen und
- R²: für Wasserstoff, für jeweils geradkettiges oder verzweigtes Alkyl mit 1 bis 10 Kohlenstoffatomen, Halogenalkyl mit 1 bis 6 Kohlenstoffatomen und 1 bis 13 gleichen oder verschiedenen Halogenatomen oder Alkoxyalkyl mit jeweils 1 bis 6 Kohlenstoffatomen in den einzelnen Alkylteilen steht oder
- R¹ und R²: gemeinsam mit dem Kohlenstoffatom, an welches sie gebunden sind, für jeweils gegebenenfalls einfach bis vierfach, gleich oder verschieden substituiertes, gesättigtes oder ungesättigtes Cycloalkyl oder Heterocyclyl mit jeweils 3 bis 8 Kohlenstoffatomen und gegebenenfalls 1 oder 2 gleichen oder verschiedenen Heteroatomen - insbesondere Stickstoff, Sauerstoff und/oder Schwefel - stehen, wobei als Substituenten jeweils infrage kommen:
Fluor, Chlor, Brom, geradkettiges oder verzweigtes Alkyl mit 1 bis 4 Kohlenstoffatomen, geradkettiges oder verzweigtes Alkanoyl mit 1 bis 5 Kohlenstoffatomen, geradkettiges oder verzweigtes Alkandiyl mit 3 bis 6 Kohlenstoffatomen, geradkettiges oder verzweigtes Dioxyalkylen mit 1 bis 6 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Alkylthio mit 1 bis 4 Kohlenstoffatomen, Cycloalkyl mit 3 bis 7 Kohlenstoffatomen oder Phenyl,
- R³: für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht,
- R⁴: für Wasserstoff, für Fluor, Chlor, Brom, für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für geradkettiges oder verzweigtes Alkoxy mit 1 bis 6 Kohlenstoffatomen steht,
- R⁵: für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht und
- n: für eine Zahl 0, 1 oder 2 steht.

Ganz besonders bevorzugt sind Verbindungen der Formel (I), bei welchen
- R¹: für Wasserstoff für jeweils geradkettiges oder verzweigtes Alkyl mit 1 bis 8 Kohlenstoffatomen, Alkenyl mit 3 bis 5 Kohlenstoffatomen, Halogenalkyl mit 1 bis 4 Kohlenstoffatomen und 1 bis 9 gleichen oder verschiedenen Halogenatomen, Alkoxyalkyl mit jeweils 1 bis 3 Kohlenstoffatomen in den einzelnen Alkylteilen, Alkoxyalkoxyalkyl mit jeweils 1 bis 3 Kohlenstoffatomen in den einzelnen Alkylteilen, Alkylthioalkyl mit jeweils 1 bis 3 Kohlenstoffatomen in den einzelnen Alkylteilen, Cycloalkyl mit 3 bis 6 Kohlenstoffatomen, gesättigtes Heterocyclyl mit 3, 5 oder 6 Ringgliedern und 1 Heteroatom - insbesondere Stickstoff, Sauerstoff oder Schwefel - oder für jeweils gegebenenfalls im Phenyl-bzw. Heteroarylteil einfach bis dreifach, gleich oder verschieden substituiertes Phenylalkyl oder Phenyl mit gegebenenfalls 1 bis 3 Kohlenstoffatomen im Alkylteil oder Heteroaryl - insbesondere Pyridyl, Imidazolyl, Pyrazolyl, Triazolyl oder Thiazolyl - steht, wobei als Phenyl- bzw. Heteroarylsubstituenten jeweils infrage kommen:
Fluor, Chlor, Brom, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
- R²: für Wasserstoff, für jeweils geradkettiges oder verzweigtes Alkyl mit 1 bis 6 Kohlenstoffatomen, Halogenalkyl mit 1 bis 4 Kohlenstoffatomen und 1 bis 9 gleichen oder verschiedenen Halogenatomen oder Alkoxyalkyl mit jeweils 1 bis 4 Kohlenstoffatomen in den einzelnen Alkylteilen steht oder
- R¹ und R²: gemeinsam mit dem Kohlenstoffatom, an welches sie gebunden sind, für gegebenenfalls einfach bis dreifach, gleich oder verschieden substituiertes, gesättigtes oder ungesättigtes Cycloalkyl oder Heterocyclyl jeweils mit 3, 5, 6, 7 oder 8 Kohlenstoffatomen und gegebenenfalls 1 oder 2 gleichen oder verschiedenen Heteroatomen - insbesondere Stickstoff, Sauerstoff und/oder Schwefel - stehen, wobei als Substituenten jeweils infrage kommen:
Fluor, Chlor, Brom, geradkettiges oder verzweigtes Alkyl mit 1 bis 4 Kohlenstoffatomen, geradkettiges oder verzweigtes Alkanoyl mit 1 bis 4 Kohlenstoffatomen, Dioxyalkylen mit 1 bis 3 Kohlenstoffatomen, Halogenalkyl mit 1 oder 2 Kohlenstoffatomen und 1 bis 5 gleichen oder verschiedenen Halogenatomen, geradkettiges oder verzweigtes Alkoxy mit 1 oder 2 Kohlenstoffatomen, geradkettiges oder verzweigtes Alkylthio mit 1 oder 2 Kohlenstoffatomen, Cycloalkyl mit 3, 5 oder 6 Kohlenstoffatomen oder Phenyl,
- R³: für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht,
- R⁴: für Wasserstoff, für Fluor, Chlor, Brom, für geradkettiges oder verzweigtes Alkyl mit 1 bis 4 Kohlenstoffatomen, für Halogenalkyl mit 1 oder 2 Kohlenstoffatomen und 1 bis 5 gleichen oder verschiedenen Halogenatomen oder für geradkettiges oder verzweigtes Alkoxy mit 1 bis 3 Kohlenstoffatomen steht,
- R⁵: für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht und
- n: für eine Zahl 0 oder 1 steht.

Im einzelnen sei auf die als Herstellungsbeispiele genannten Verbindungen verwiesen.

Verwendet man beispielsweise 2-Amino-2-methyl-butyronitril und 2-(2,4-Dichlorphenyl)-essigsäurechlorid als Ausgangsverbindungen, so läßt sich der Reaktionsablauf des erfindungsgemäßen Verfahrens durch das folgende Formelschema darstellen:

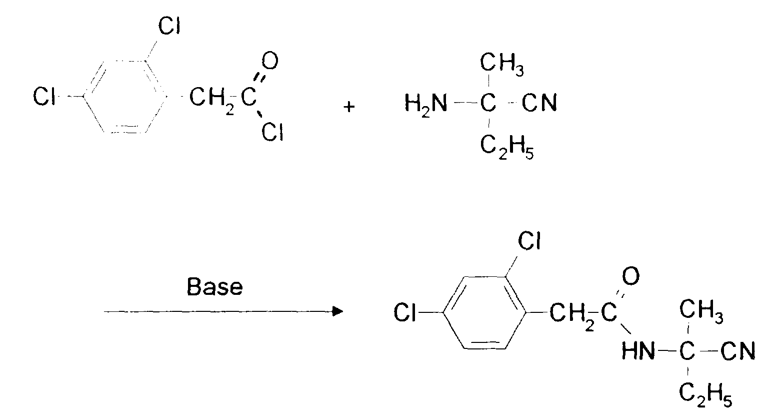

Die zur Durchführung des erfindungsgemäßen Verfahrens als Ausgangsstoffe benötigten α-Aminonitrile sind durch die Formel (II) allgemein definiert. In dieser Formel (II) stehen R¹ und R² vorzugsweise für diejenigen Reste, die bereits im Zusammenhang mit der Beschreibung der erfindungsgemäßen Verbindungen der Formel (I) als bevorzugt für diese Substituenten genannt wurden. Die α-Aminonitrile der Formel (II) sind bekannt oder erhältlich in Analogie zu bekannten Verfahren (vergl. z.B. US 4.041.045; Liebigs Ann. Chem. 764, 69-93 [1972]; EP 427 907; US 4.041.045; US 3.422.132; Can. J. Chem. 53, 3339-3350 [1975]; J. Amer. Chem. Soc. 94, 968-972 [1972]).

Die zur Durchführung des erfindungsgemäßen Verfahren weiterhin als Ausgangsprodukte erforderlichen Phenylessigsäurehalogenide sind durch die Formel (III) allgemein definiert. In dieser Formel (III) stehen R³, R⁴, R⁵ und n vorzugsweise für diejenigen Reste und Indices, die bereits im Zusammenhang mit der Beschreibung der erfindungsgemäßen Stoffe der Formel (I) als bevorzugt für diese Substituenten und diesen Index genannt wurden. Hal steht vorzugsweise für Fluor, Chlor, Brom oder Iod, insbesondere für Chlor oder Brom.
Die Phenylessigsäurehalogenide der Formel (III) sind allgemein bekannt oder erhältlich in Analogie zu bekannten Verfahren (vergl. z.B. J. Amer. Chem. Soc. 95, 3340 [1973]).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Sulfoxide, wie Dimethylsulfoxid oder Sulfolan.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, -acetate, -carbonate oder -hydrogencarbonate, wie beispielsweise Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie insbesondere auch tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist auch möglich, das als Reaktionspartner eingesetzte α-Aminnonitril der Formel (II) in einem entsprechenden Überschuß gleichzeitig als Säurebindemittel einzusetzen.

Die Reaktionstemperaturen können bei der Durchführung des erfindungsgemäßen Verfahrens in einem größeren Bereich variiert werden. Im allgemeinen arbeitet man bei Temperaturen zwischen -20°C und 150°C, vorzugsweise bei Temperaturen zwischen 0°C und 120°C.

Das erfindungsgemäße Verfahren wird üblicherweise unter Normaldruck durchgeführt Es ist jedoch auch möglich unter erhöhtem oder vermindertem Druck zu arbeiten.

Zur Durchführung des erfindungsgemäßen Verfahrens setzt man pro Mol an α-Antinonitril der Formel (II) im allgemeinen 0,1 bis 5,0 Mol, vorzugsweise 0,5 bis 2,0 Mol an Phenylessigsäurehalogenid der Formel (III) und gegebenenfalls 0,5 bis 3,0 Mol, vorzugsweise 1,0 bis 2,0 Mol an Reaktionshilfsmittel ein. Die Reaktionsdurchführung, Aufarbeitung und Isolierung der Reaktionsprodukte erfolgt in Analogie zu bekannten Verfahren (vergl. hierzu beispielsweise J. Org. Chem. 43, 2576-2581 [1978] oder die Herstellungsbeispiele).

Die erfindungsgemäßen N-Phenylacetaminonitrile der Formel (I) sind geeignete Zwischenprodukte zur Herstellung von insektiziden, akariziden und herbiziden 3-Arylpyrrolidin-2,4-dionen der Formel (VI), 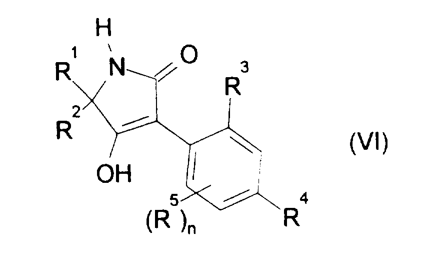 in welcher
R¹, R², R³, R⁴, R⁵ und n die oben angegebene Bedeutung haben,
indem man sie zunächst in einer ersten Stufe mit Alkoholen der Formel (IV),

R-OH (IV)

in welcher
R für Alkyl steht,
gegebenenfalls in Gegenwart eines Verdünnungsmittels und in Gegenwart von Schwefelsäure als Reaktionshilfsmittels umsetzt und dann die so erhältlichen N-Phenylacetaminocarbonsäureester der Formel (V), 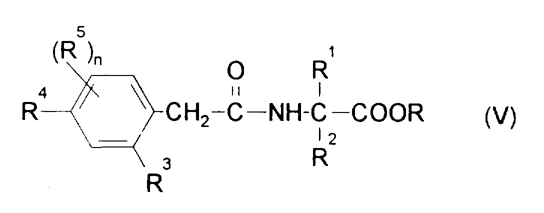 in welcher
R, R¹, R², R³, R⁴, R⁵ und ndie oben angegebene Bedeutung haben,
in einer anschließenden 2.Stufe gegebenenfalls in Gegenwart eines Verdünnungsmittels und gegebenenfalls in Gegenwart eines Reaktionshilfsmittels cyclisiert.

Verwendet man beispielsweise 2-(2,4-Dichlorphenyl)-N-(1-cyano-2-butyl)-acetamid und Methanol als Ausgangsverbindungen, so läßt sich der Reaktionsablauf der weiteren Umsetzung der erfindungsgemäßen Verbindungen der Formel (I) durch das folgende Formelschema darstellen:

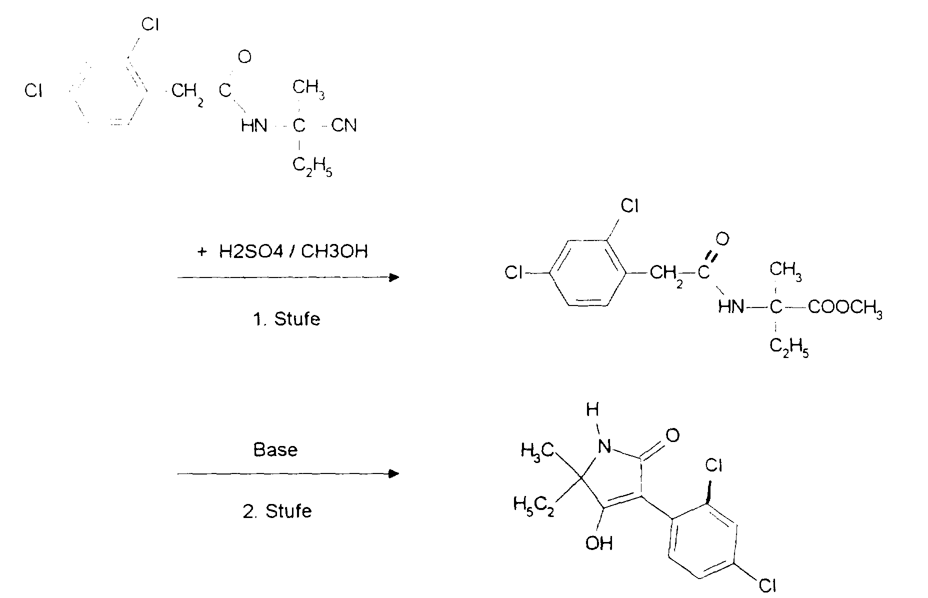

Die zur Durchführung der weiteren Umsetzung der erfindungsgemäßen Verbindungen der Formel (I) als Ausgangsprodukte benötigten Alkohole sind durch die Formel (IV) allgemein definiert. In dieser Formel (IV) steht R vorzugsweise für geradkettiges oder verzweigtes Alkyl mit 1 bis 6, insbesondere 1 bis 4 Kohlenstoffatomen, besonders bevorzugt für Methyl oder Ethyl. Die Alkohole der Formel (IV) sind allgemein bekannte Verbindungen der organischen Chemie.

Als Reaktionshilfsmittel zur Durchführung der 1. Stufe der weiteren Umsetzung der erfindungsgemäßen Verbindungen der Formel (I) kommt insbesondere konzentrierte Schwefelsäure infrage. Gegebenenfalls ist es auch möglich, stark acide organische Säuren, wie beispielsweise Phenylsulfonsäure, Toluolsulfonsäure, Methansulfonsäure oder Trifluoressigsäure zu verwenden.

Als Verdünnungsmittel zur Durchführung der 1. Stufe der weiteren Umsetzung der erfindungsgemäßen Verbindungen der Formel (I) kommen inerte organische Lösungsmittel infrage. Hierzu gehören insbesondere aliphatische, alicyclische oder aromatische,gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff oder Ether, wie Diethylether, Diisopropylether, Dioxan, Tetrahydrofuran oder Ethylenglykoldimethyl- oder -diethylether.

Die 1. Stufe der weiteren Umsetzung der erfindungsgemäßen Verbindungen der Formel (I) wird üblicherweise unter Normaldruck durchgeführt. Es ist jedoch auch möglich unter erhöhtem oder vermindertem Druck zu arbeiten.

Die Reaktionstemperaturen können bei der Durchführung der 1. Stufe der weiteren Umsetzung der erfindungsgemäßen Verbindungen der Formel (I) in einem größeren Bereich variiert werden. Im allgemeinen arbeitet man bei Temperaturen zwischen - 20°C und 150°C, vorzugsweise bei Temperaturen zwischen 0 C und 120 C.

Zur Durchführung der 1. Stufe der weiteren Umsetzung der erfindungsgemäßen Verbindungen der Formel (I) setzt man pro Mol an N-Phenylacetaminonitril der Formel (I) im allgemeinen 1,0 bis 5,0 Mol, vorzugsweise 1,0 bis 2,5 Mol an Alkohol der Formel (IV) und gegebenenfalls 1,0 bis 5,0 Mol, vorzugsweise 1,0 bis 2,5 Mol an als Reaktionshilfsmittel verwendeter Säure ein.
Die Reaktionsdurchführung, Aufarbeitung und Isolierung der Reaktionsprodukte erfolgt in Analogie zu bekannten Verfahren (vergl. hierzu auch die Herstellungsbeispiele).

Die mit Hilfe der 1. Stufe der weiteren Umsetzung der erfindungsgemäßen Verbindungen der Formel (I) erhältlichen N-Phenylacetaminocarbonsäureester der Formel (V) sind teilweise bekannt (vergl. z.B. EP 456 063; JP 49011415).

Noch nicht bekannt und ebenfalls Gegenstand der Erfindung sind N-Phenylacetaminocarbonsäureester der Formel (Va), 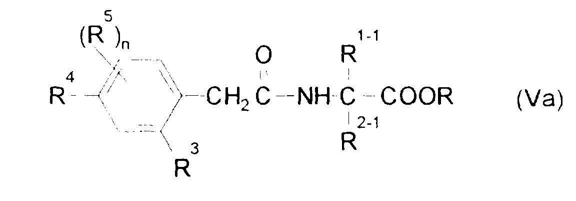 in welcher
- R: für Alkyl steht,
- R¹⁻¹: für Wasserstoff oder für jeweils gegebenenfalls substituiertes Alkyl, Alkenyl, Cycloalkyl, Aryl oder Heterocyclyl steht und
- R²⁻¹: für Halogenalkyl steht oder
- R¹⁻¹ und R²⁻¹: gemeinsam mit dem Kohlenstoffatom, an welches sie gebunden sind, für jeweils substituiertes Cycloalkyl oder Heterocyclyl stehen und
- R³: für Halogen, Alkyl oder Alkoxy steht,
- R⁴: für Wasserstoff, Halogen, Alkyl, Halogenalkyl oder Alkoxy steht,
- R⁵: für Halogen, Alkyl oder Alkoxy steht und
- n: für eine Zahl 0, 1, 2 oder 3 steht.

Bevorzugt sind Verbindungen der Formel (Va), bei welchen
- R: für C₁-C₆-Alkyl steht,
- R¹⁻¹: für Wasserstoff, für jeweils geradkettiges oder verzweigtes Alkyl mit 1 bis 12 Kohlenstoffatomen, Alkenyl mit 3 bis 8 Kohlenstoffatomen, Halogenalkyl mit 1 bis 8 Kohlenstoffatomen und 1 bis 17 gleichen oder verschiedenen Halogenatomen, Alkoxyalkyl mit jeweils 1 bis 8 Kohlenstoffatomen in den einzelnen Alkylteilen, Alkoxyalkoxyalkyl mit jeweils 1 bis 8 Kohlenstoffatomen in den einzelnen Alkylteilen, Alkylthioalkyl mit jeweils 1 bis 8 Kohlenstoffatomen in den einzelnen Alkylteilen, Cycloalkyl mit 3 bis 8 Kohlenstoffatomen, gesättigtes Heterocyclyl mit 3 bis 8 Ringgliedern und 1 bis 3 gleichen oder verschiedenen Heteroatomen - insbesondere Stickstoff, Sauerstoff und/oder Schwefel - oder für jeweils gegebenenfalls im Aryl- bzw. Heteroarylteil einfach oder mehrfach. gleich oder verschieden substituiertes Arylalkyl oder Aryl mit jeweils 6 bis 10 Kohlenstoffatomen im Arylteil und gegebenenfalls 1 bis 6 Kohlenstoffatomen im geradkettigen oder verzweigten Alkylteil oder Heteroaryl mit 2 bis 9 Kohlenstoffatomen und 1 bis 4 gleichen oder verschiedenen Heteroatomen - insbesondere Stickstoff Sauerstoff und/oder Schwefel - steht, wobei als Aryl- bzw Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen sowie jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen und
- R²⁻¹: für Halogenalkyl mit 1 bis 8 Kohlenstoffatomen und 1 bis 17 gleichen oder verschiedenen Halogenatomen steht oder
- R¹⁻¹ und R²⁻¹: gemeinsam mit dem Kohlenstoffatom, an welches sie gebunden sind, für jeweils einfach oder mehrfach, gleich oder verschieden substituiertes, gesättigtes oder ungesättigtes Cycloalkyl oder Heterocyclyl mit 3 bis 12 Kohlenstoffatomen und gegebenenfalls 1 bis 3 gleichen oder verschiedenen Heteroatomen - insbesondere Stickstoff, Sauerstoff und/oder Schwefel - stehen, wobei als Substituenten jeweils infrage kommen:
Halogen, geradkettiges oder verzweigtes Alkyl mit 1 bis 6 Kohlenstoffatomen, geradkettiges oder verzweigtes Alkandiyl mit 3 bis 8 Kohlenstoffatomen, geradkettiges oder verzweigtes Dioxyalkylen mit 1 bis 8 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxy mit 1 bis 6 Kohlenstoffatomen, geradkettiges oder verzweigtes Alkylthio mit 1 bis 6 Kohlenstoffatomen, Cycloalkyl mit 3 bis 8 Kohlenstoffatomen, Aryl mit 6 bis 10 Kohlenstoffatomen,
- R³: für Fluor, Chlor, Brom, Iod, für geradkettiges oder verzweigtes Alkyl mit 1 bis 8 Kohlenstoffatomen oder für geradkettiges oder verzweigtes Alkoxy mit 1 bis 8 Kohlenstoffatomen steht,
- R⁴: für Wasserstoff, für Fluor, Chlor, Brom, lod, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für geradkettiges oder verzweigtes Alkoxy mit 1 bis 8 Kohlenstoffatomen steht,
- R⁵: für Fluor, Chlor, Brom, Iod, für geradkettiges oder verzweigtes Alkyl mit 1 bis 8 Kohlenstoffatomen oder für geradkettiges oder verzweigtes Alkoxy mit 1 bis 8 Kohlenstoffatomen steht und
- n: für eine Zahl 0, 1, 2 oder 3 steht.

Besonders bevorzugt sind Verbindungen der Formel (Va), bei welchen
- R: für C₁₋₄-Alkyl steht,
- R¹⁻¹: für Wasserstoff, für jeweils geradkettiges oder verzweigtes Alkyl mit 1 bis 10 Kohlenstoffatomen, Alkenyl mit 3 bis 6 Kohlenstoffatomen, Halogenalkyl mit 1 bis 6 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, Alkoxyalkoxyalkyl mit jeweils 1 bis 6 Kohlenstoffatomen in den einzelnen Alkylteilen, Alkylthioalkyl mit jeweils 1 bis 6 Kohlenstoffatomen in den einzelnen Alkylteilen, Cycloalkyl mit 3 bis 7 Kohlenstoffatomen, gesättigtes Heterocyclyl mit 3 bis 7 Ringgliedern und 1 oder 2 gleichen oder verschiedenen Heteroatomen - insbesondere Stickstoff, Sauerstoff und/oder Schwefel - oder für jeweils gegebenenfalls im Aryl- bzw. Heteroarylteil einfach bis funffach, gleich oder verschieden substituiertes Arylalkyl oder Aryl mit jeweils 6 oder 10 Kohlenstoffatomen im Arylteil und gegebenenfalls 1 bis 4 Kohlenstoffatomen im geradkettigen oder verzweigten Alkylteil oder Heteroaryl mit 2 bis 9 Kohlenstoffatomen und 1 bis 3 gleichen oder verschiedenen Heteroatomen - insbesondere Stickstoff, Sauerstoff und/oder Schwefel - steht, wobei als Aryl- bzw Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen sowie jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen und
- R²⁻¹: für Halogenalkyl mit 1 bis 6 Kohlenstoffatomen und 1 bis 13 gleichen oder verschiedenen Halogenatomen steht oder
- R¹⁻¹ und R²⁻¹: gemeinsam mit dem Kohlenstoffatom, an welches sie gebunden sind, für jeweils einfach bis vierfach, gleich oder verschieden substituiertes, gesättigtes oder ungesättigtes Cycloalkyl oder Heterocyclyl mit 3 bis 8 Kohlenstoffatomen und gegebenenfalls 1 oder 2 gleichen oder verschiedenen Heteroatomen - insbesondere Stickstoff Sauerstoff und/oder Schwefel - stehen, wobei als Substituenten jeweils infrage kommen:
Fluor, Chlor, Brom, geradkettiges oder verzweigtes Alkyl mit 1 bis 4 Kohlenstoffatomen, geradkettiges oder verzweigtes Alkandiyl mit 3 bis 6 Kohlenstoffatomen, geradkettiges oder verzweigtes Dioxyalkylen mit 1 bis 6 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Alkylthio mit 1 bis 4 Kohlenstoffatomen, Cycloalkyl mit 3 bis 7 Kohlenstoffatomen oder Phenyl,
- R³: für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht,
- R⁴: für Wasserstoff für Fluor, Chlor, Brom, für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für geradkettiges oder verzweigtes Alkoxy mit 1 bis 6 Kohlenstoffatomen steht,
- R⁵: für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht und
- n: für eine Zahl 0, 1 oder 2 steht.

Ganz besonders bevorzugt sind Verbindungen der Formel (Va), bei welchen
- R: für Methyl, Ethyl, Propyl, i-Propyl steht,
- R¹⁻¹: für Wasserstoff, für jeweils geradkettiges oder verzweigtes Alkyl mit 1 bis 8 Kohlenstoffatomen, Alkenyl mit 3 bis 5 Kohlenstoffatomen, Halogenalkyl mit 1 bis 4 Kohlenstoffatomen und 1 bis 9 gleichen oder verschiedenen Halogenatomen, Alkoxyalkyl mit jeweils 1 bis 3 Kohlenstoffatomen in den einzelnen Alkylteilen, Alkoxyalkoxyalkyl mit jeweils 1 bis 3 Kohlenstoffatomen in den einzelnen Alkylteilen, Alkylthioalkyl mit jeweils 1 bis 3 Kohlenstoffatomen in den einzelnen Alkylteilen, Cycloalkyl mit 3 bis 6 Kohlenstoffatomen, gesättigtes Heterocyclyl mit 3, 5 oder 6 Ringgliedern und 1 Heteroatom - insbesondere Stickstoff, Sauerstoff oder Schwefel - oder für jeweils gegebenenfalls im Phenyl- bzw. Heteroarylteil einfach bis dreifach, gleich oder verschieden substituiertes Phenylalkyl oder Phenyl mit gegebenenfalls 1 bis 3 Kohlenstoffatomen im Alkylteil oder Heteroaryl - insbesondere Pyridyl, Imidazolyl, Pyrazolyl, Triazolyl oder Thiazolyl - steht, wobei als Phenyl- bzw. Heteroarylsubstituenten jeweils infrage kommen:
Fluor, Chlor, Brom, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
- R²⁻¹: für Halogenalkyl mit 1 bis 4 Kohlenstoffatomen und 1 bis 9 gleichen oder verschiedenen Halogenatomen steht oder
- R¹⁻¹ und R²⁻¹: gemeinsam mit dem Kohlenstoffatom, an welches sie gebunden sind, für einfach bis dreifach, gleich oder verschieden substituiertes. gesättigtes oder ungesättigtes Cycloalkyl mit 3, 5, 6, 7 oder 8 Kohlenstoffatomen stehen, wobei als Substituenten infrage kommen:
Fluor, Chlor, Brom, geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, geradkettiges oder verzweigtes Alkoxy mit 1 oder 2 Kohlenstoffatomen, geradkettiges oder verzweigtes Alkylthio mit 1 oder 2 Kohlenstoffatomen, Cycloalkyl mit 3, 5 oder 6 Kohlenstoffatomen oder Phenyl,
- R³: für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht,
- R⁴: für Wasserstoff, für Fluor, Chlor, Brom, für geradkettiges oder verzweigtes Alkyl mit 1 bis 4 Kohlenstoffatomen, für Halogenalkyl mit 1 oder 2 Kohlenstoffatomen und 1 bis 5 gleichen oder verschiedenen Halogenatomen oder für geradkettiges oder verzweigtes Alkoxy mit 1 bis 3 Kohlenstoffatomen steht,
- R⁵: für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht und
- n: für eine Zahl 0 oder 1 steht.

Als Verdünnungsmittel zur Durchführung der 2. Stufe der weiteren Umsetzung der erfindungsgemäßen Verbindungen der Formel (I) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Ether, wie Diethylether, Diisopropylether, Dioxan, Tetrahydrofuran oder Ethylenglykoldimethyl- oder -diethylether; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfolan oder Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Die 2. Stufe der weiteren Umsetzung der erfindungsgemäßen Verbindungen der Formel (I) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die 2. Stufe der weiteren Umsetzung der erfindungsgemäßen Verbindungen der Formel (I) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyldimethyl-ammoniummethylsulfat, Dimethyl-C₁₂/C₁₄-alkyl-benzylammoniumchlorid, Dimethyl-C₁₂/C₁₄-alkyl-benzylammoniumbromid, Methyl-trialkyl-C₈/C₁₀-ammoniumchlorid; Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die 2 Stufe der weiteren Umsetzung der erfindungsgemäßen Verbindungen der Formel (I) wird üblicherweise unter Normaldruck durchgeführt. Es ist jedoch auch möglich unter erhöhtem oder vermindertem Druck zu arbeiten.

Die Reaktionstemperaturen können bei der Durchführung der 2. Stufe der weiteren Umsetzung der erfindungsgemäßen Verbindungen der Formel (I) in einem größeren Bereich variiert werden. Im allgemeinen arbeitet man bei Temperaturen zwischen 0°C und 250°C, vorzugsweise bei Temperaturen zwischen 20 C und 200°C.

Zur Durchführung der 2. Stufe der weiteren Umsetzung der erfindungsgemäßen Verbindungen der Formel (I) setzt man pro Mol an N-Phenylacetaminocarbonsäureester der Formel (V) im allgemeinen 1,0 bis 5,0 Mol, vorzugsweise 1,0 bis 2,5 Mol an als Reaktionshilfsmittel verwendeter Base ein.
Die Reaktionsdurchführung, Aufarbeitung und Isolierung der Reaktionsprodukte erfolgt in Analogie zu bekannten Verfahren (vergl. hierzu beispielsweise EP 456 063 oder auch die Herstellungsbeispiele).

Die Reinigung der Zwischen- und Endprodukte der einzelnen Stufen erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie (¹H-NMR).

Die mit Hilfe der erfindungsgemäßen N-Phenylacetaminonitrile der Formel (I) sowie der N-Phenylacetaminocarbonsäureester der Formel (V) erhältlichen 3-Aryl-pyrrolidin-2,4-dione der Formel (VI) sind als Insektizide, Akarizide und Herbizide entweder bekannt (vergl. z.B. EP 456 063) oder Gegenstand einer eigenen parallelen Patentanmeldung.

### Herstellungsbeispiele:

### Beipiel 1:

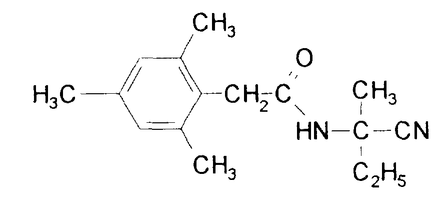

Zu 29,4 g (0,3 Mol) 2-Amino-2-methyl-butyronitril (vergl. z.B. US 4.041.045) und 42 ml (0,3 Mol) Triethylamin in 450 ml absolutem Tetrahydrofuran gibt man bei 0 C bis 10 C tropfenweise unter Rühren 58,8 g (0,3 Mol) Mesitylenessigsäurechlorid (vergl. z.B. Tetrahedron 31, 2691-2694 [1975]) in 50 ml absolutem Tetrahydrofuran und rührt nach beendeter Zugabe bei Raumtemperatur bis im Dünnschichtchromatogramm kein Ausgangsprodukt mehr nachweisbar ist. Zur Aufarbeitung gibt man die Reaktionsmischung unter Rühren in eine Mischung aus 1000 ml Eiswasser und 200 ml 1N-Salzsäure, saugt ausgefallenen Feststoff ab, löst den Rückstand in Dichlormethan, trennt die wässrige Phase ab, trocknet die organische Phase über Magnesiumsulfat und entfernt das Lösungsmittel im Vakuum.

Man erhält 69,5 g (90 % der Theorie) an 2-(2,4,6-Trimethylphenyl)-N-(2-cyano-2-butyl)-acetamid vom Schmelzpunkt 155-157°C.

In entsprechender Weise und gemäß den allgemeinen Angaben zur Herstellung erhält man die folgenden N-Phenylacetaminonitrile der allgemeinen Formel (I):

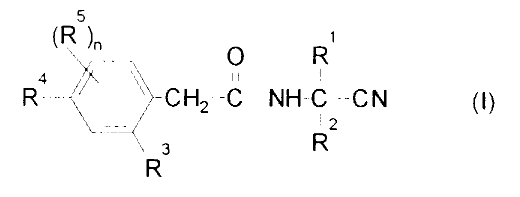

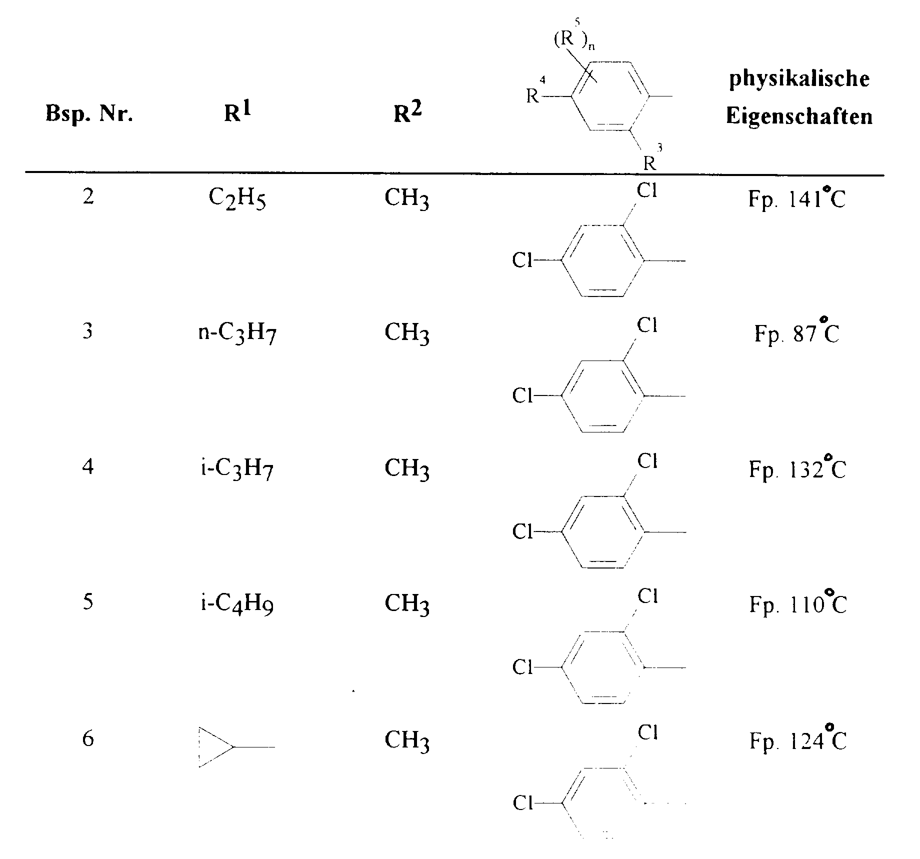

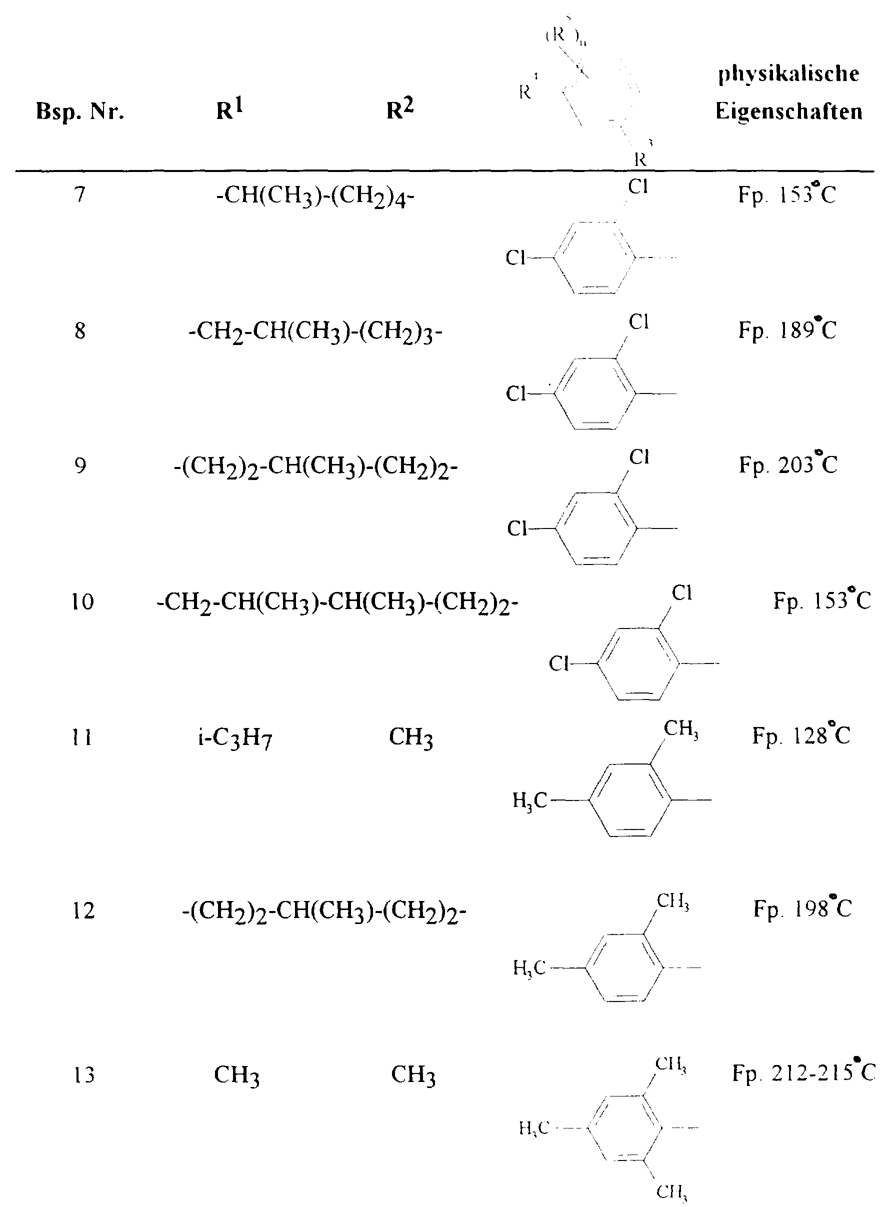

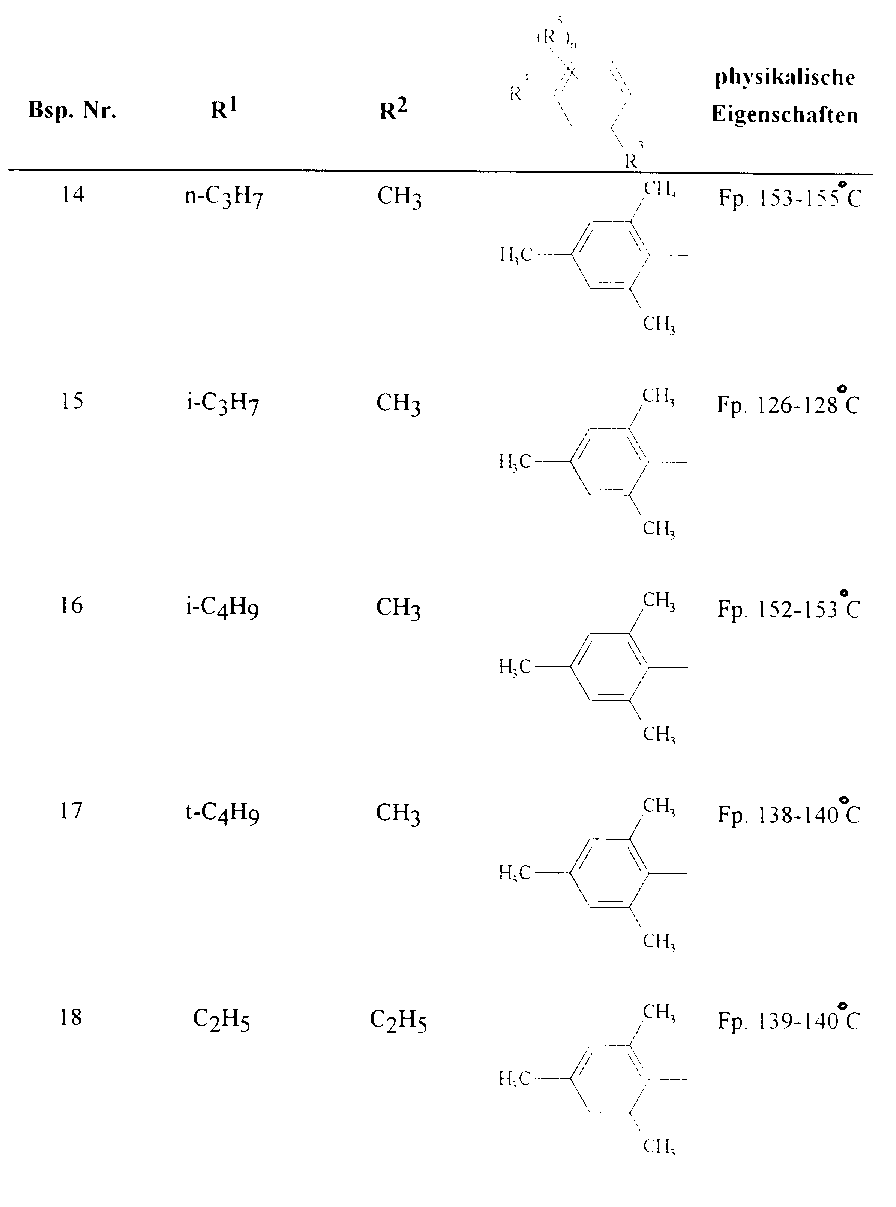

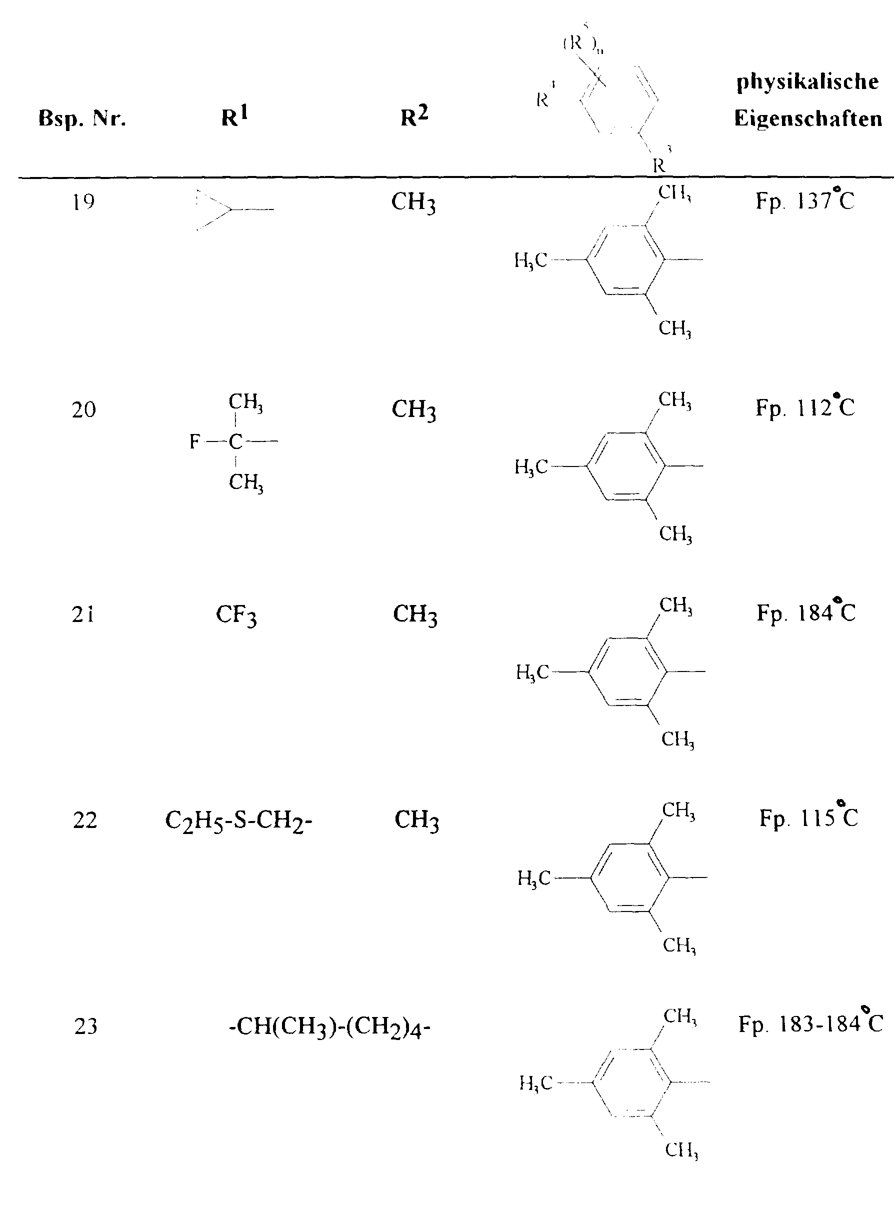

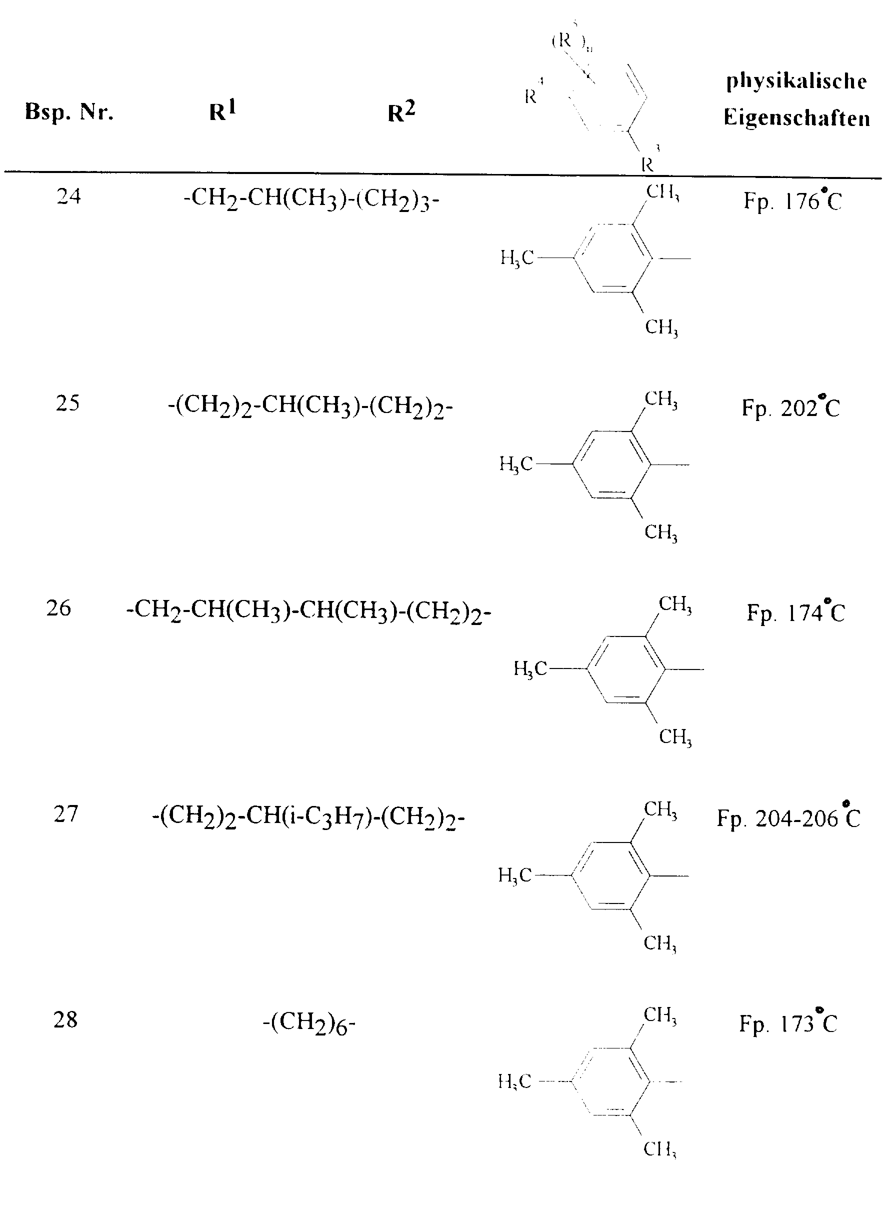

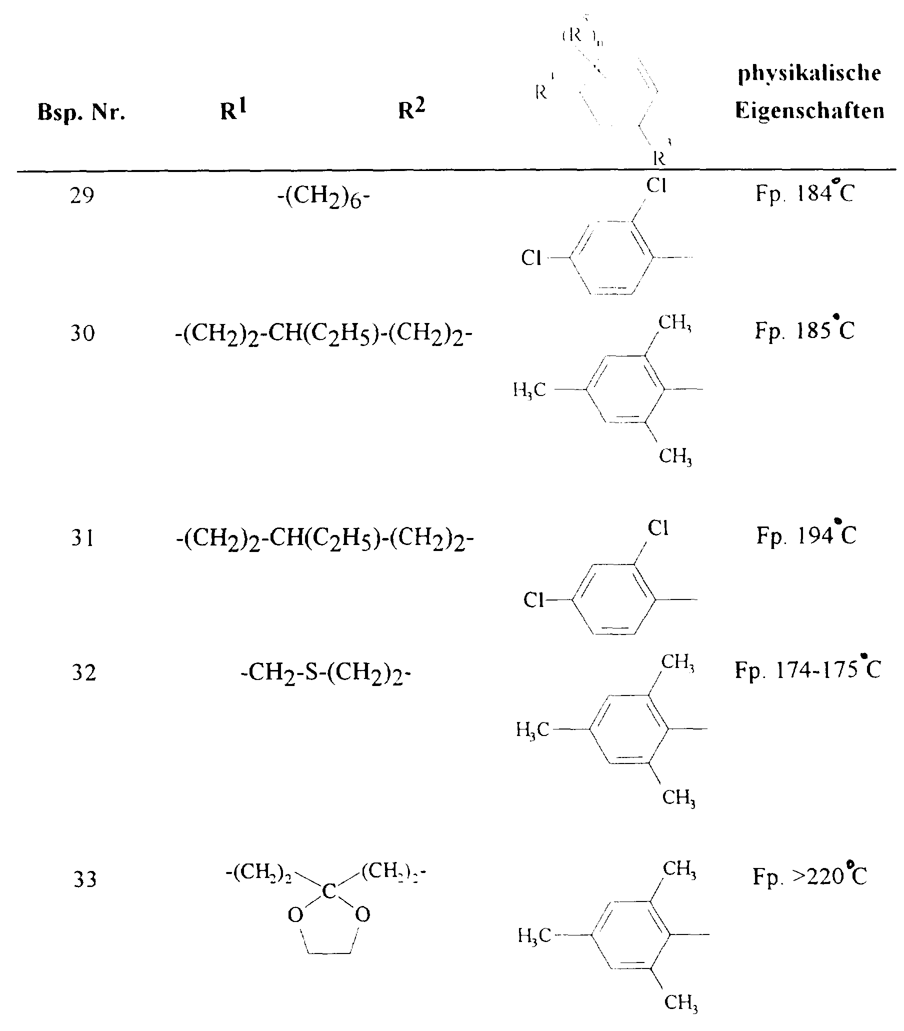

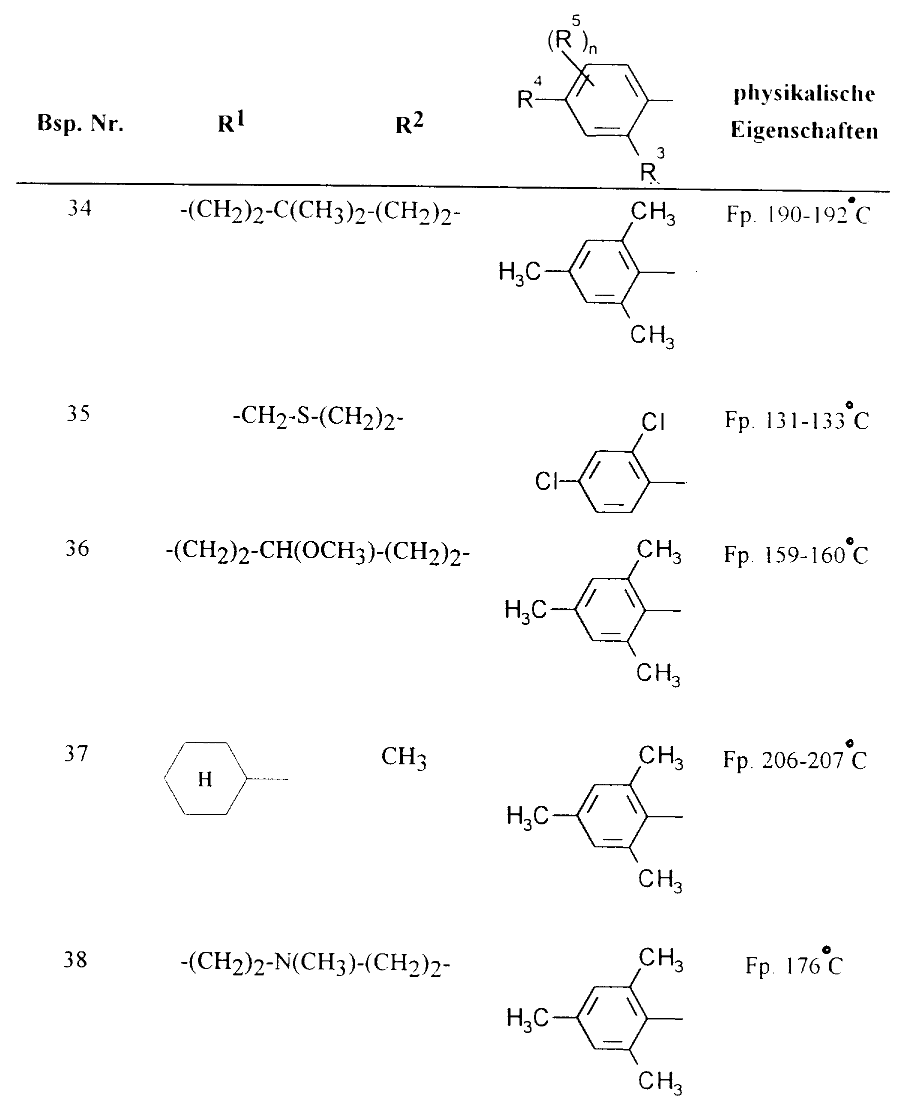

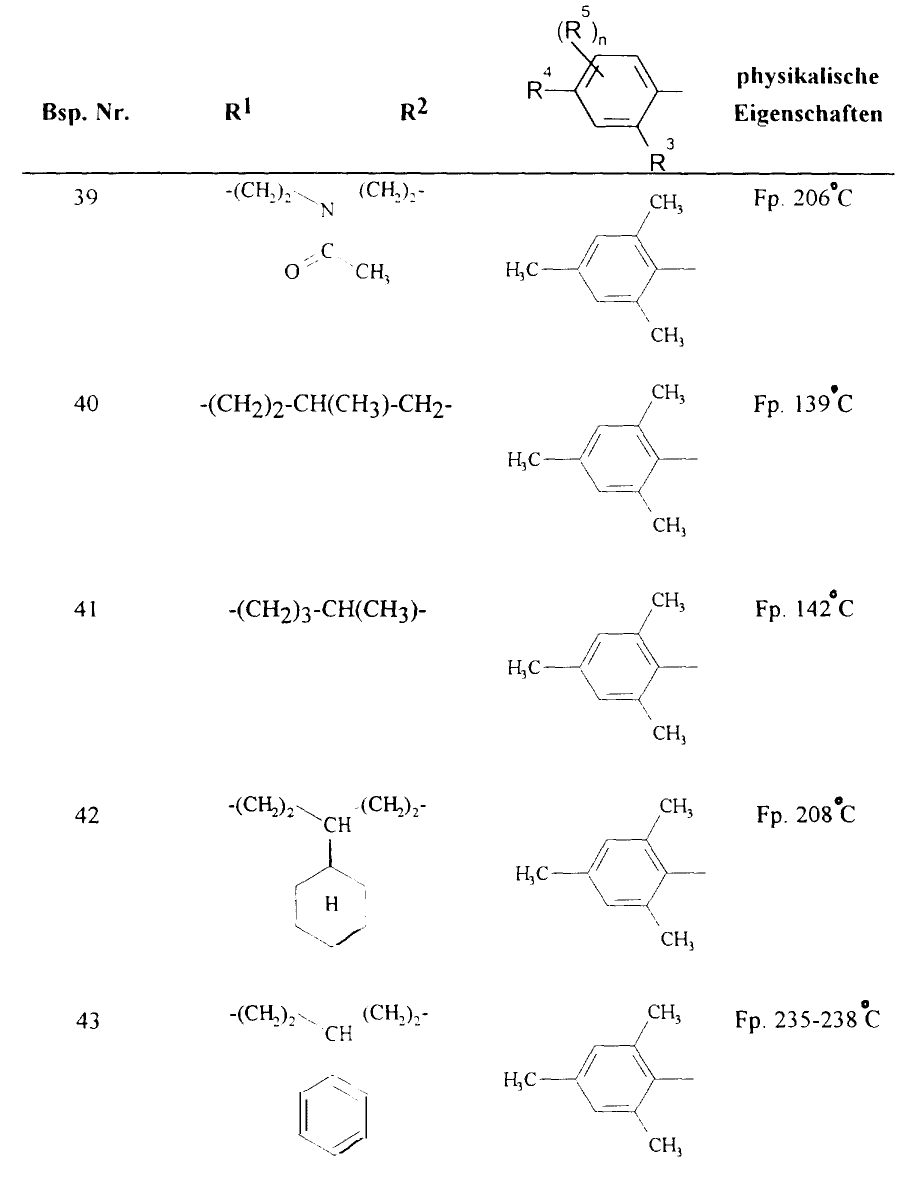

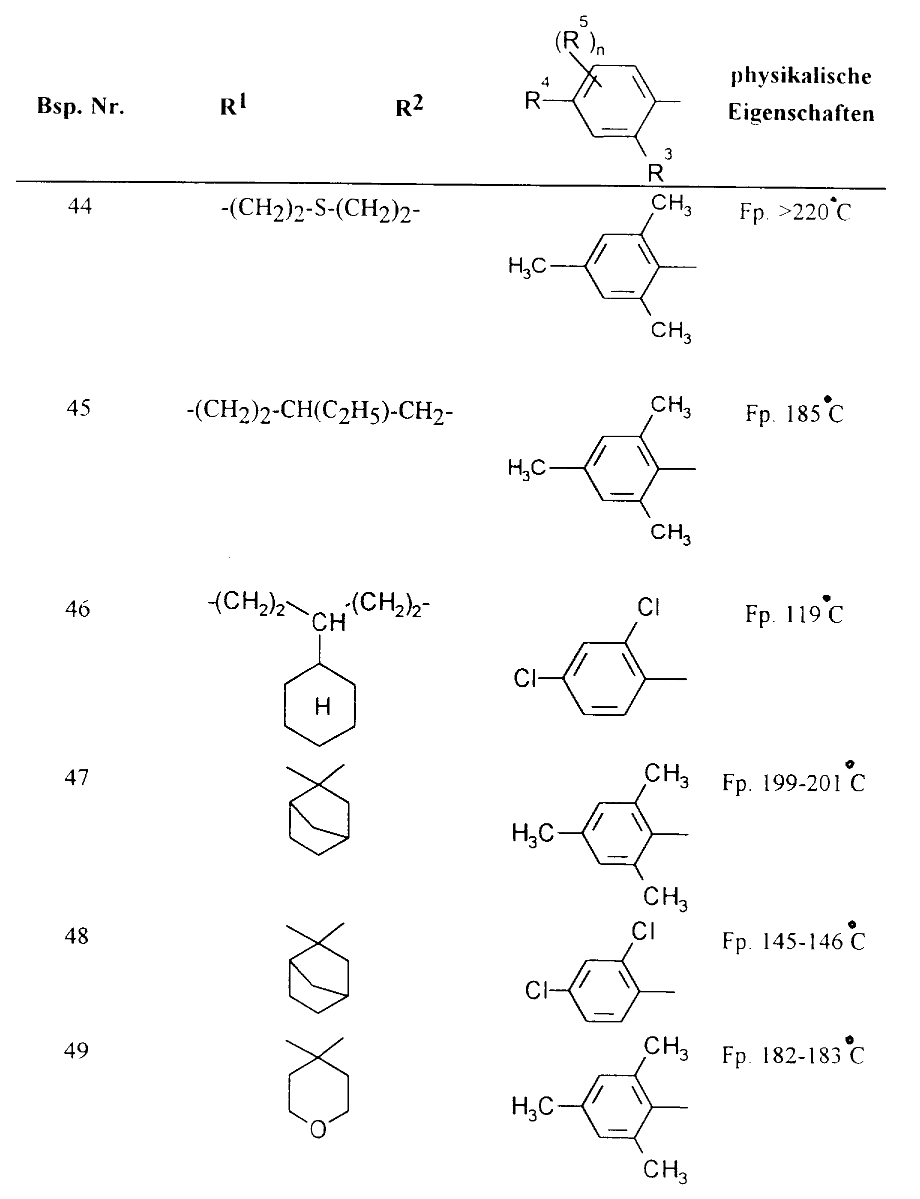

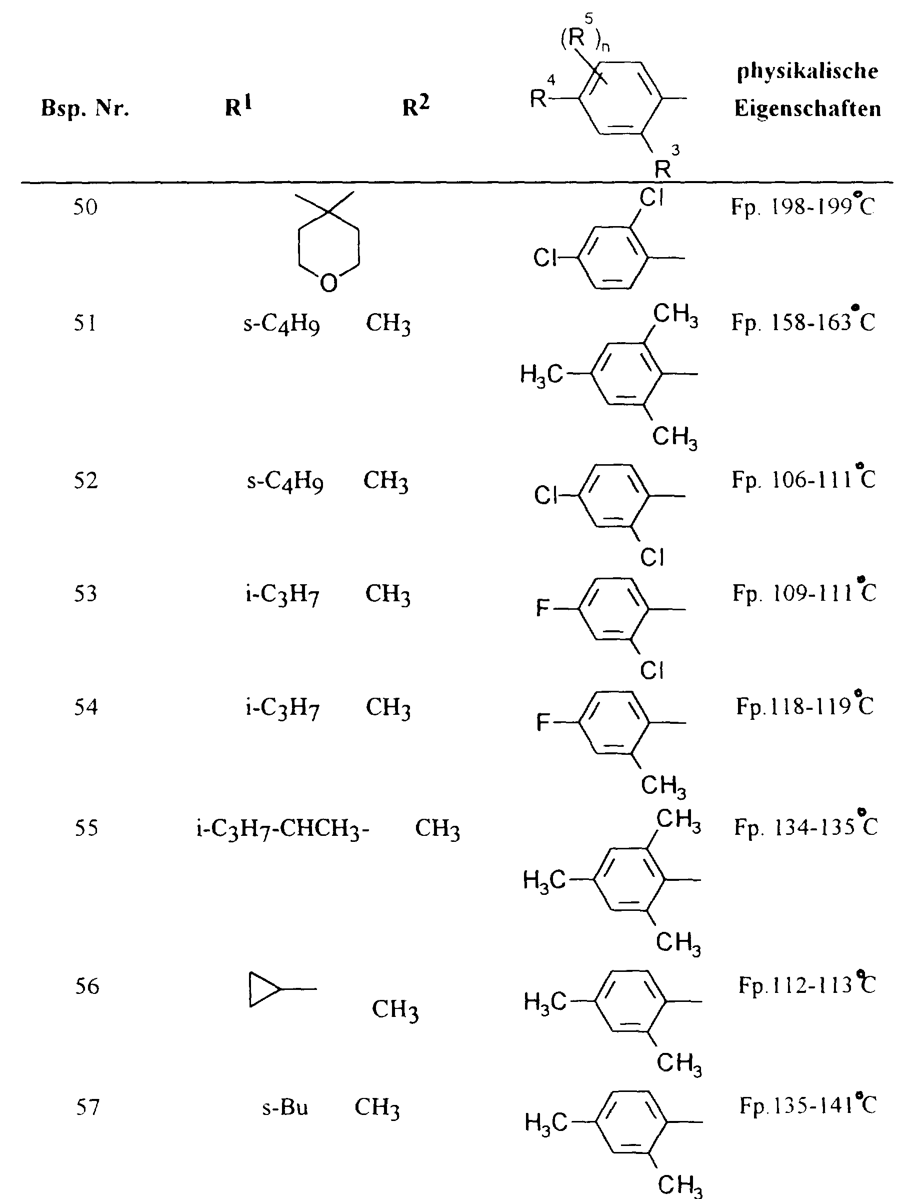

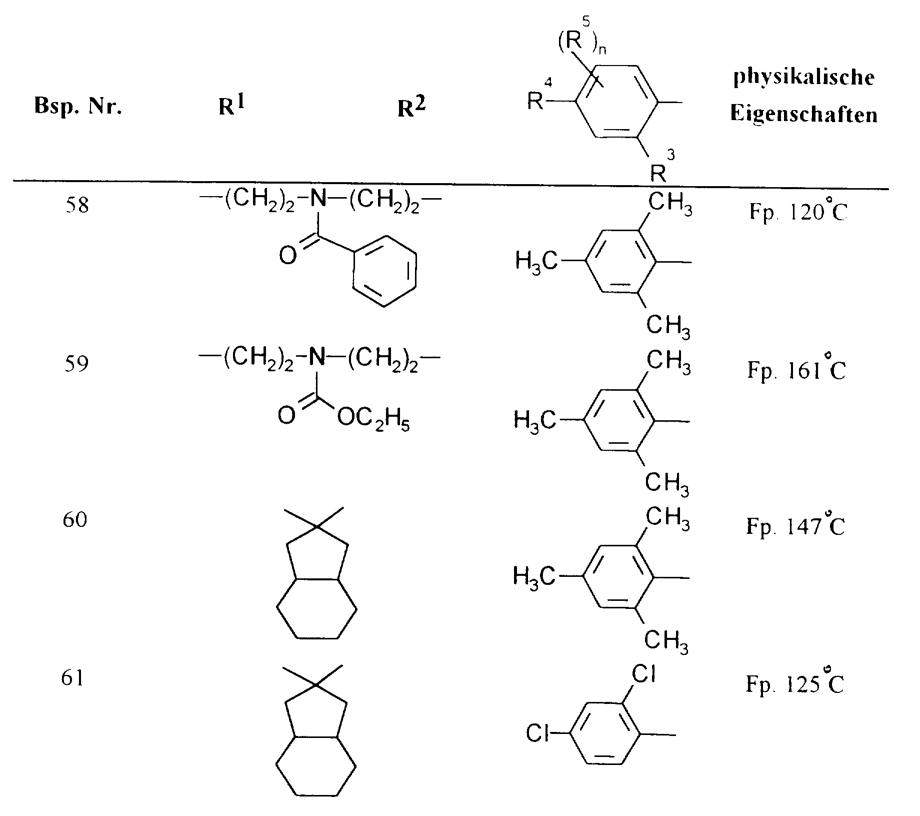

### Herstellung der Folgeprodukte:

### Beispiel V-I:

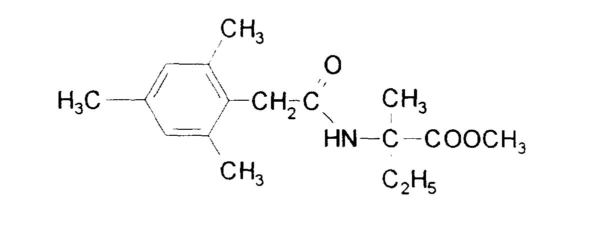

Zu 127 g (1,293 Mol) konzentrierter Schwefelsäure gibt man unter Rühren und Eiskühlung tropfenweise 66,8 g (0,259 Mol) 2-(2,4,6-Trimethylphenyl)-N-(1-cyano-2-butyl)-acetamid in 260 ml Dichlormethan gelöst, wobei sich die Temperatur der Reaktionsmischung auf 30°C bis 40°C erwärmt und rührt nach beendeter Zugabe weitere 2 Stunden bei 30°C bis 40°C bis die Dichlormethanphase der Reaktionsmischung farblos geworden ist. Anschließend gibt man ebenfalls tropfenweise unter Eiskühlung 180 ml absolutes Methanol zu, wobei sich die Reaktionsmischung abermals bis 40°C erwärmt. Anschließend rührt man weitere 6 Stunden bei 40°C bis 70°C. Zur Aufarbeitung gibt man die Reaktionsmischung unter Rühren in 1.300 g Eis, extrahiert mit Dichlormethan, wäscht die vereinigten organischen Phasen mit wässriger Natriumhydrogencarbonatlösung säurefrei, trocknet über Magnesiumsulfat und entfernt das Lösungsmittel im Vakuum.

Man erhält 62,5 g (83 % der Theorie) an 2-(2,4,6-Trimethylphenyl)-N-(1-methoxycarbonyl-2-butyl)-acetamid vom Schmelzpunkt 107-109°C.

In entsprechender Weise und gemäß den allgemeinen Angaben zur Herstellung erhält man die folgenden N-Phenylacetaminocarbonsäureester der Formel (V),

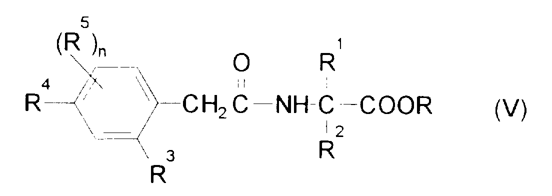

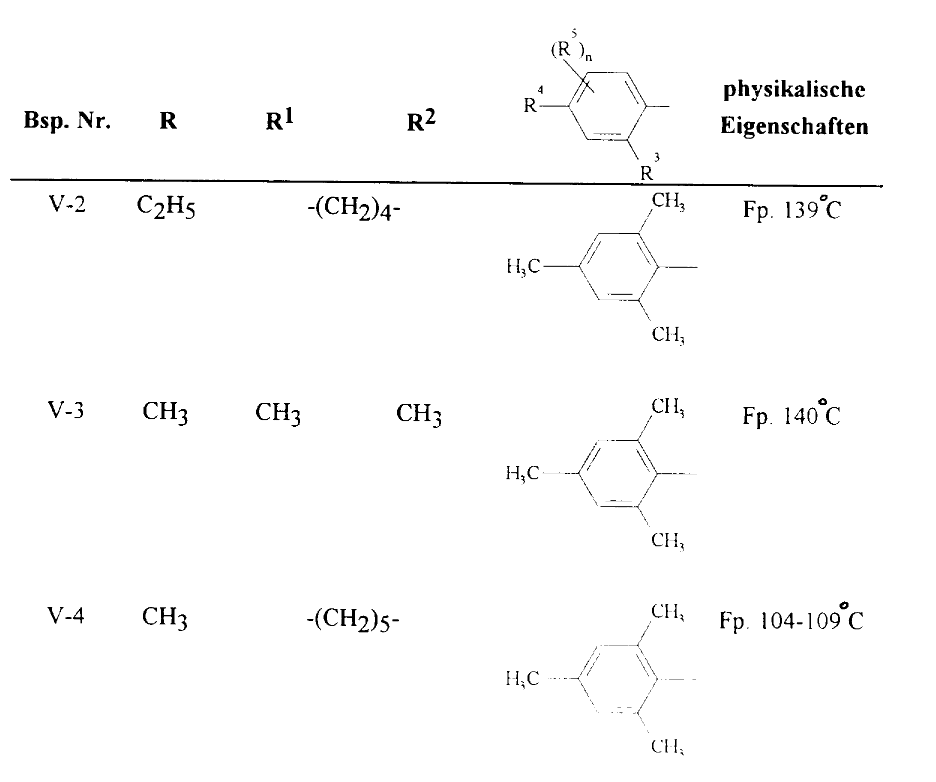

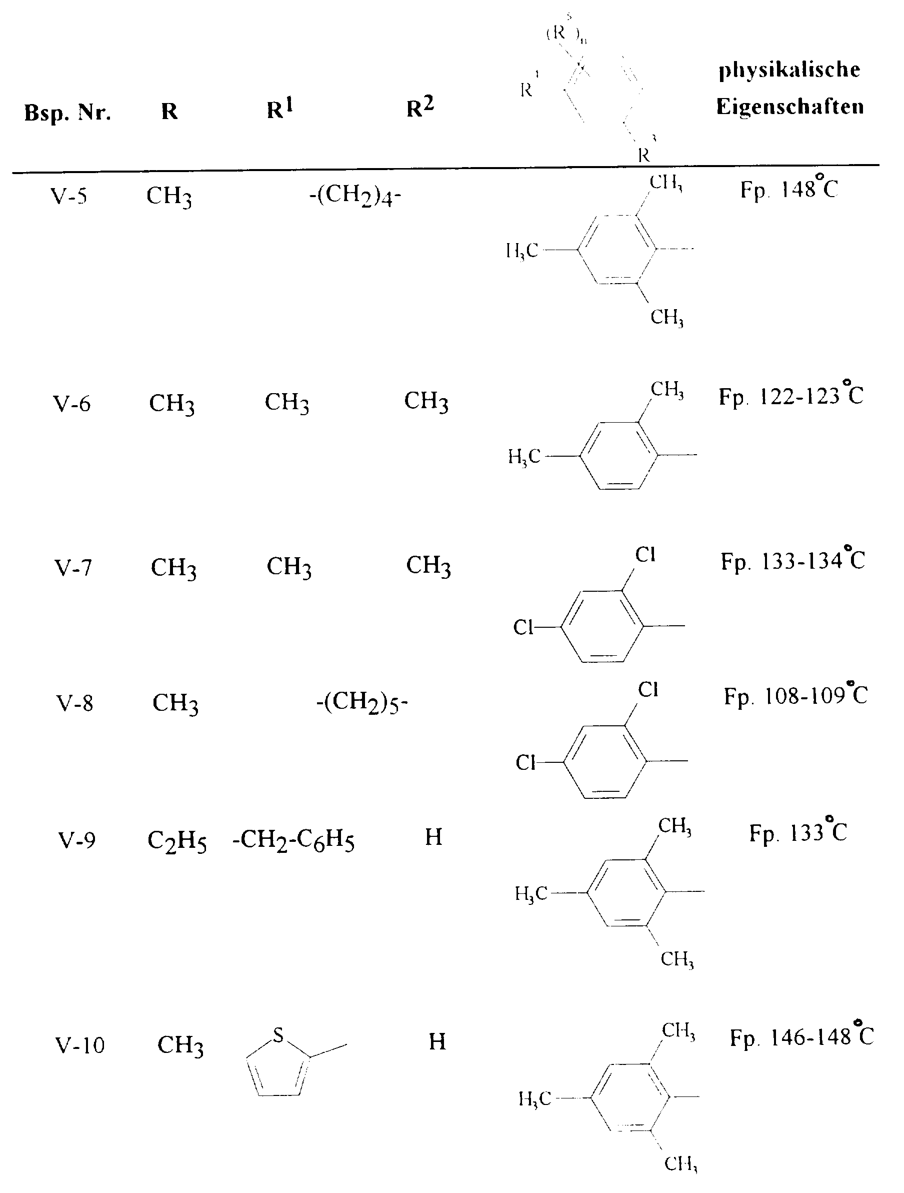

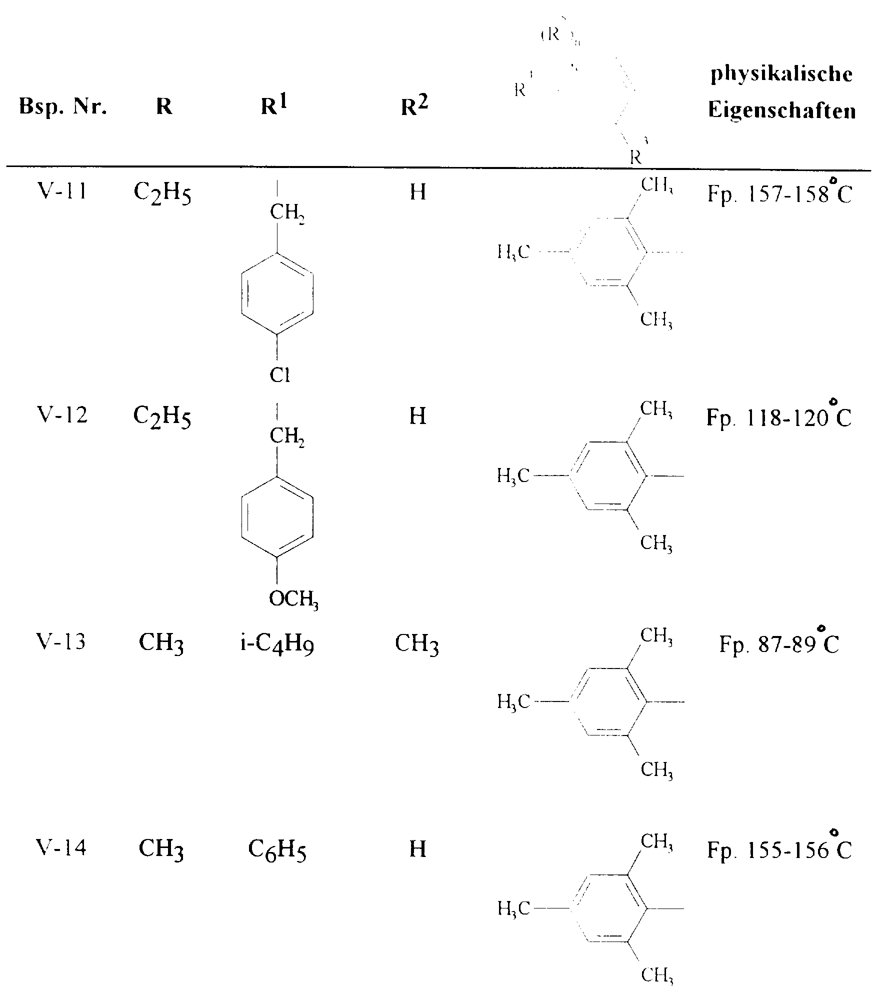

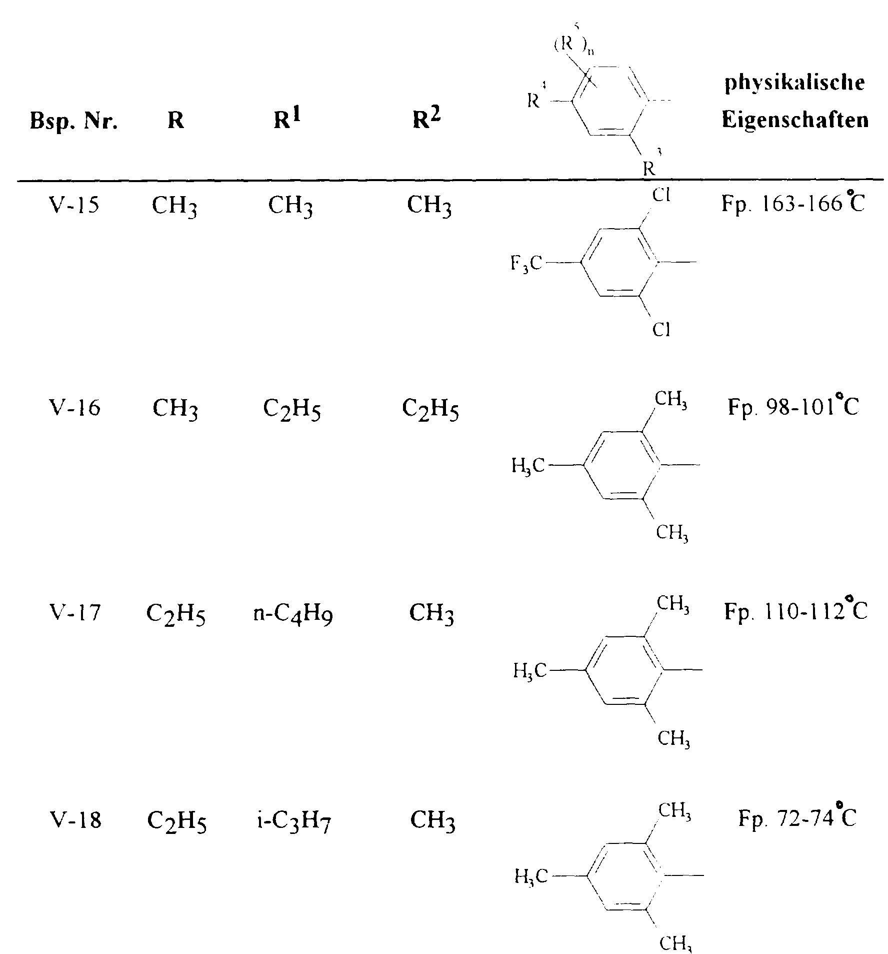

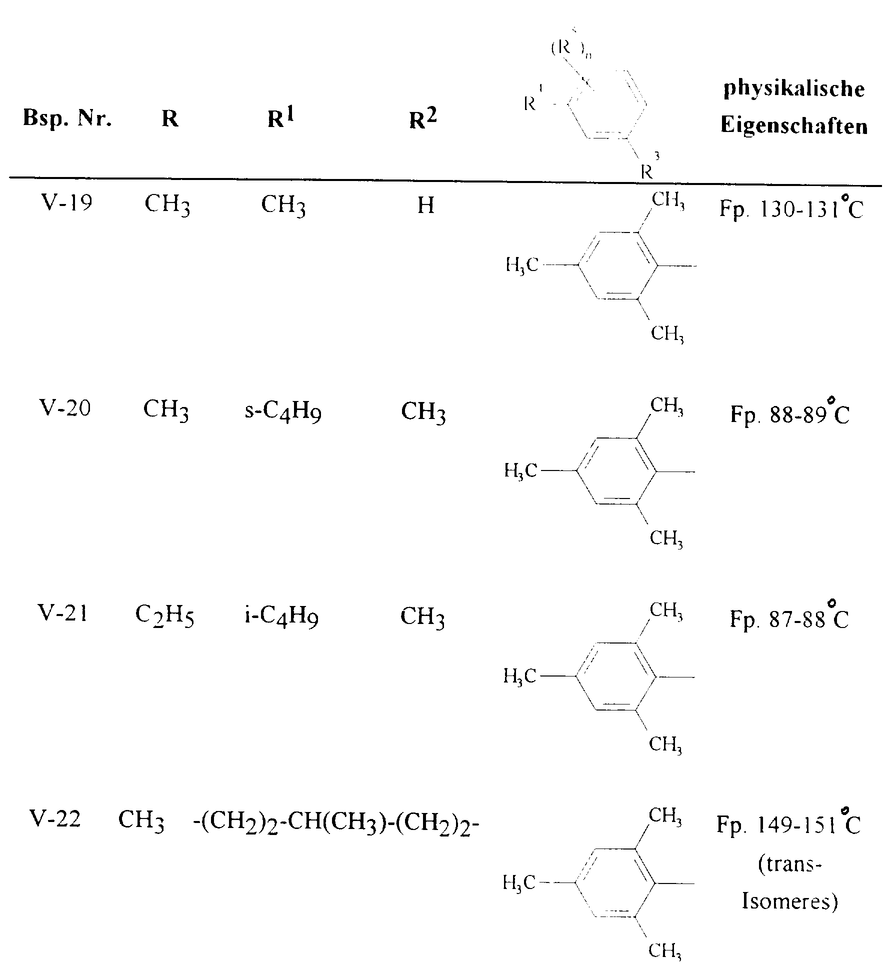

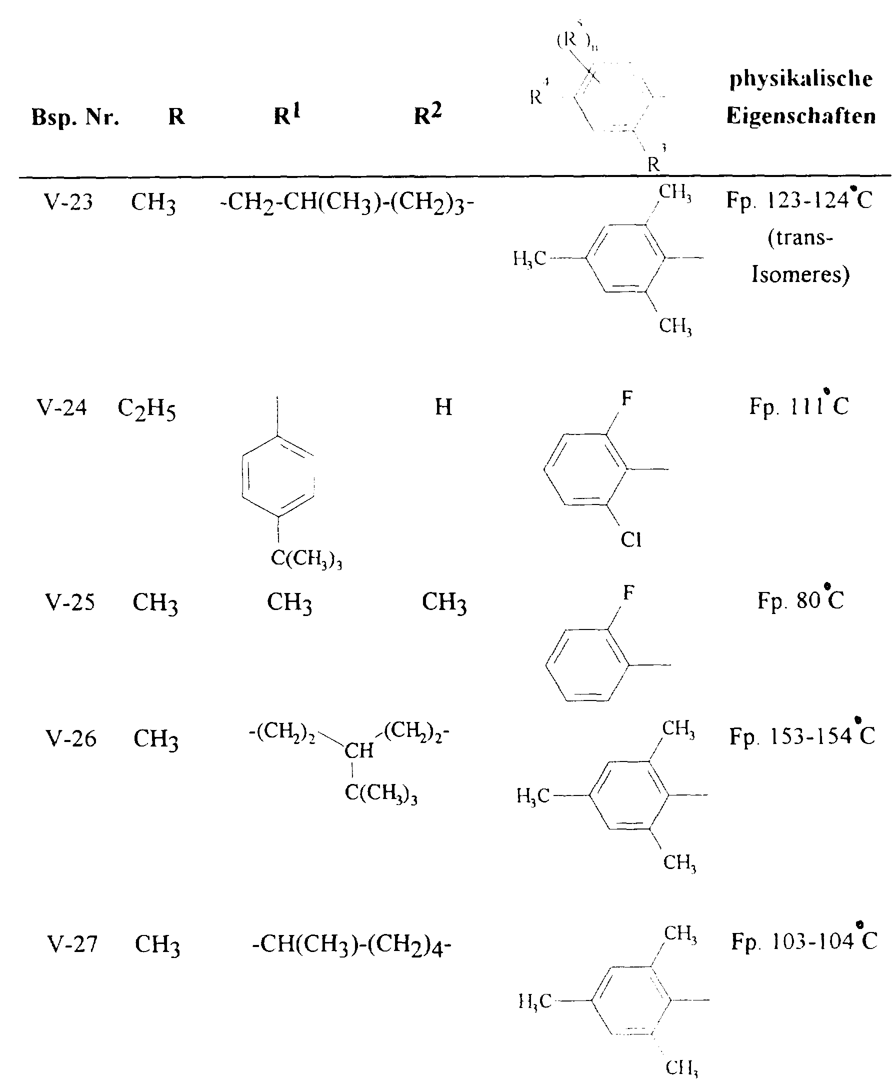

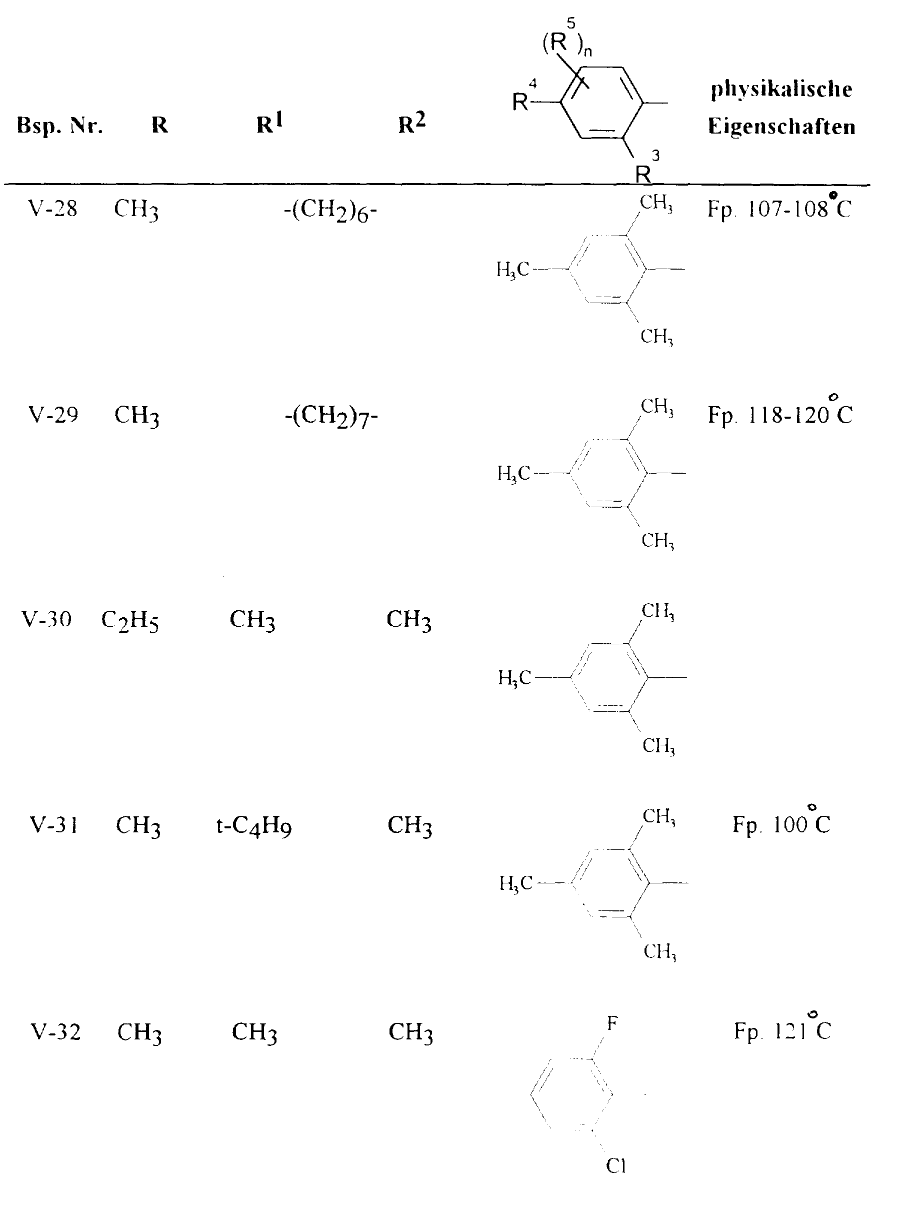

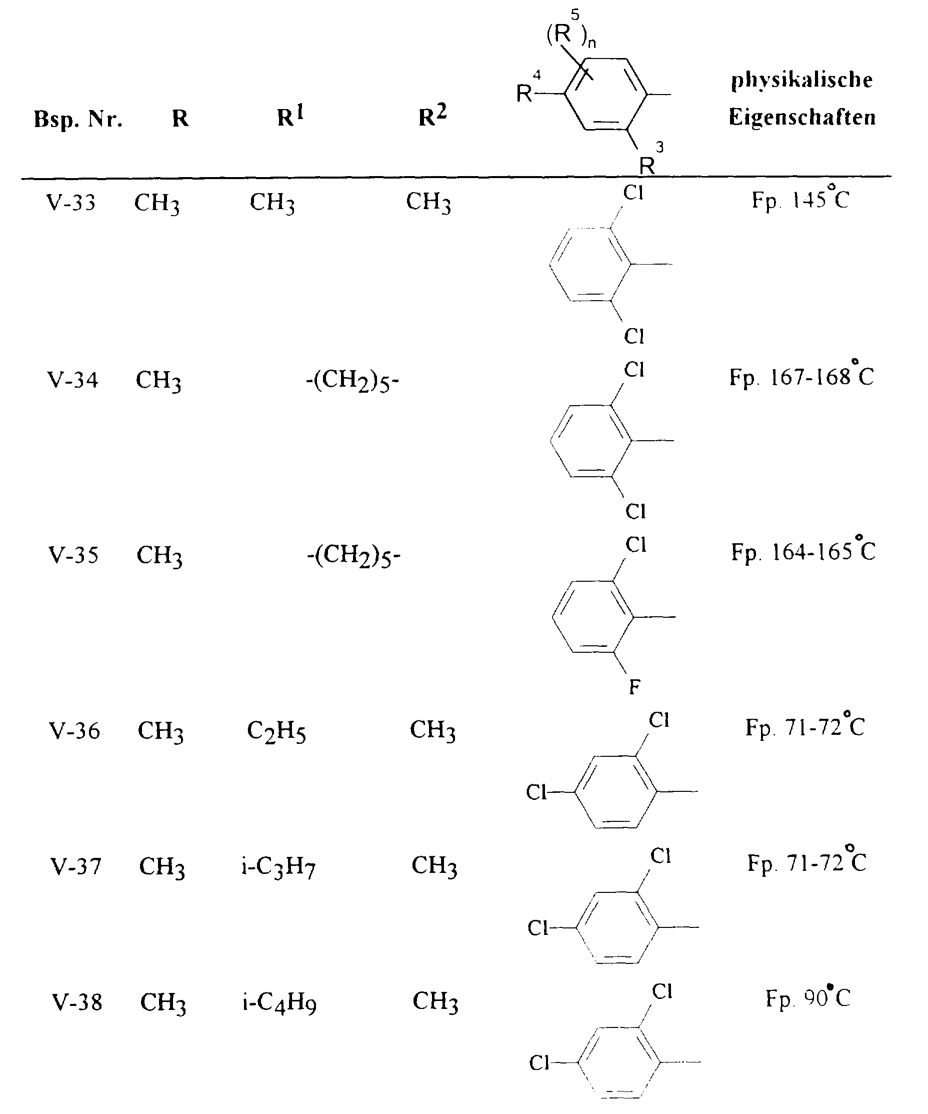

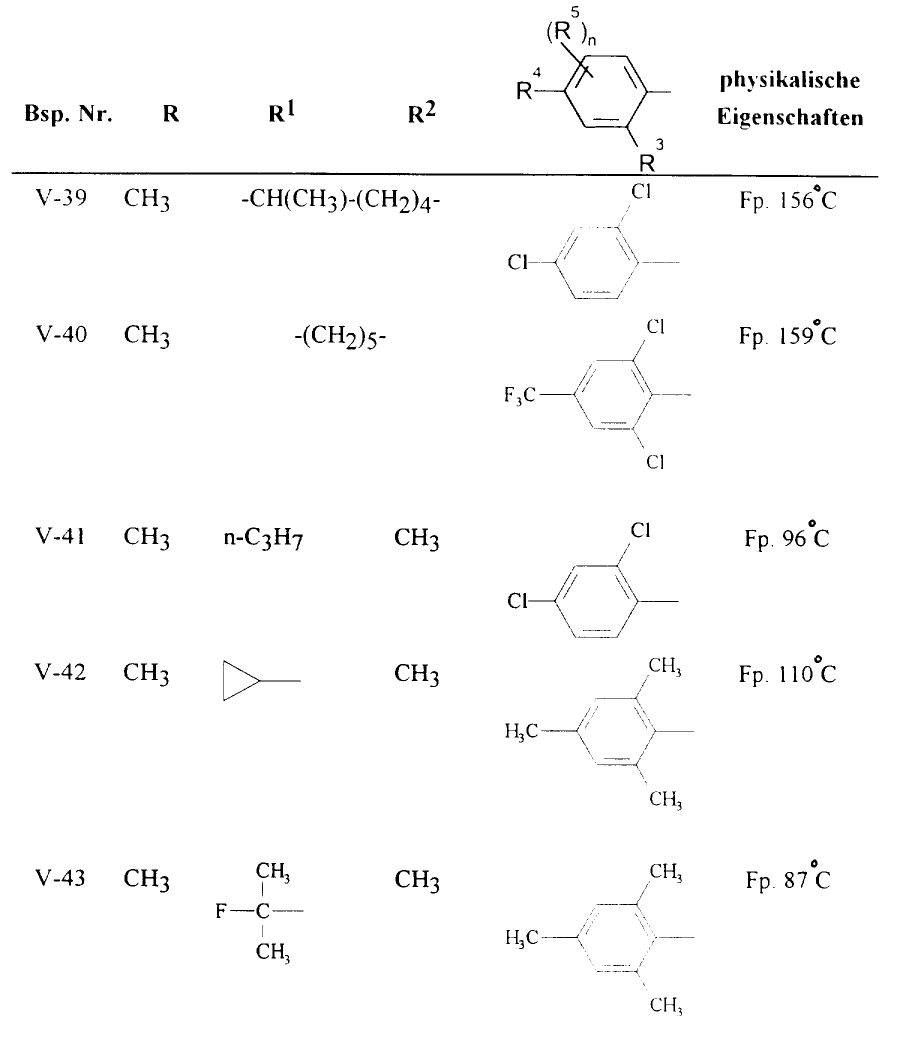

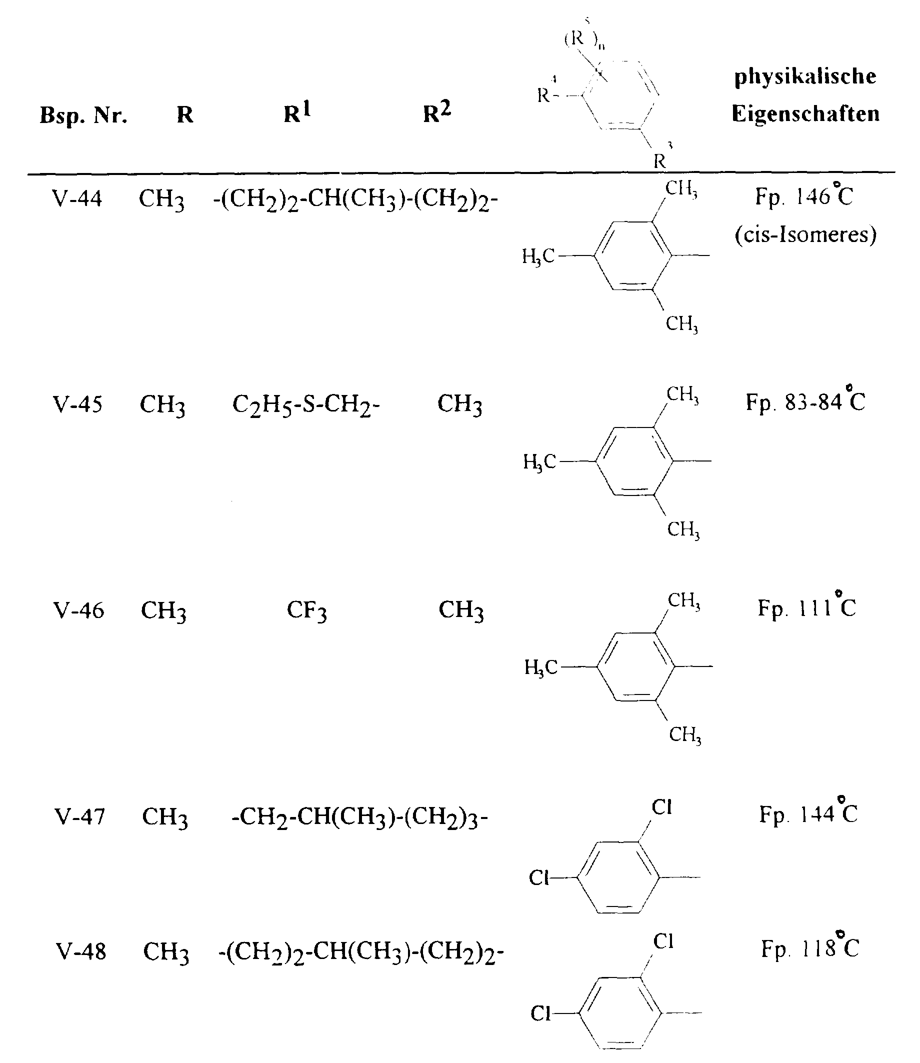

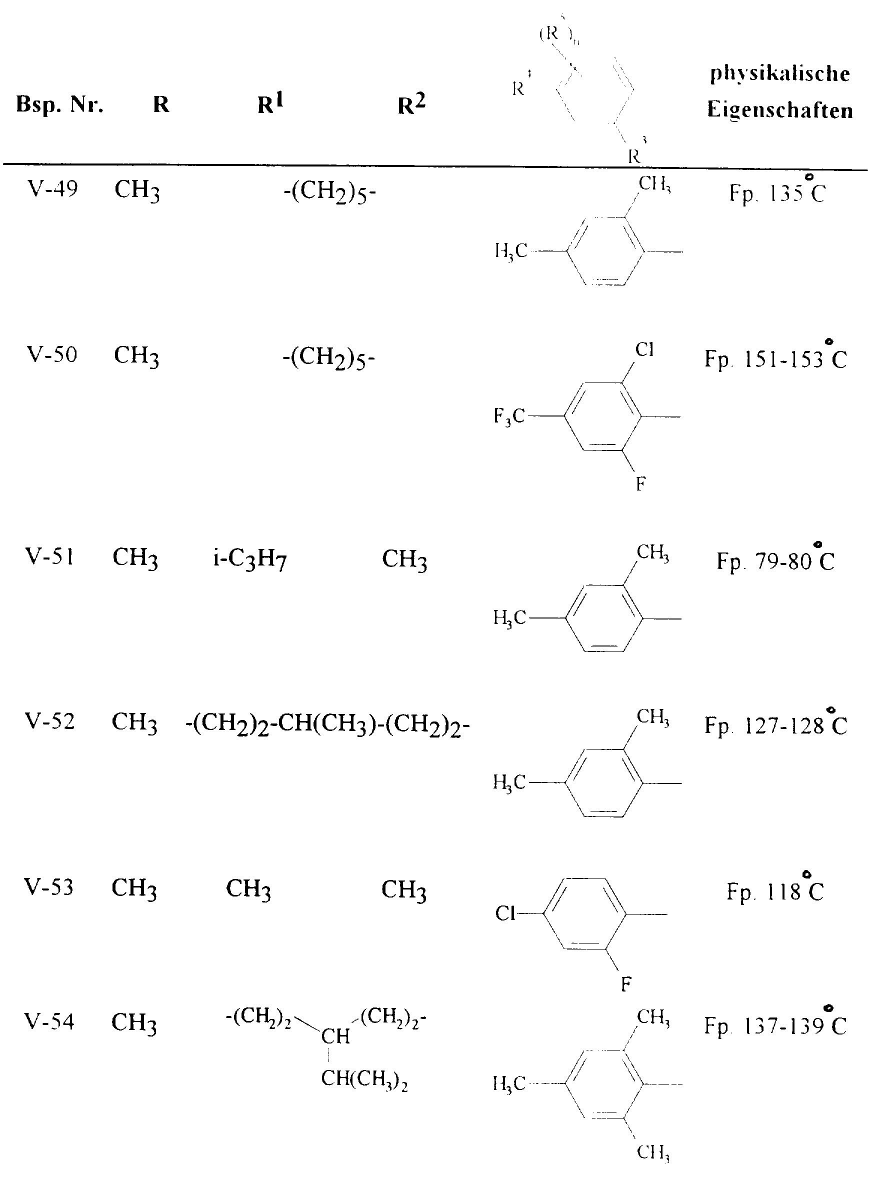

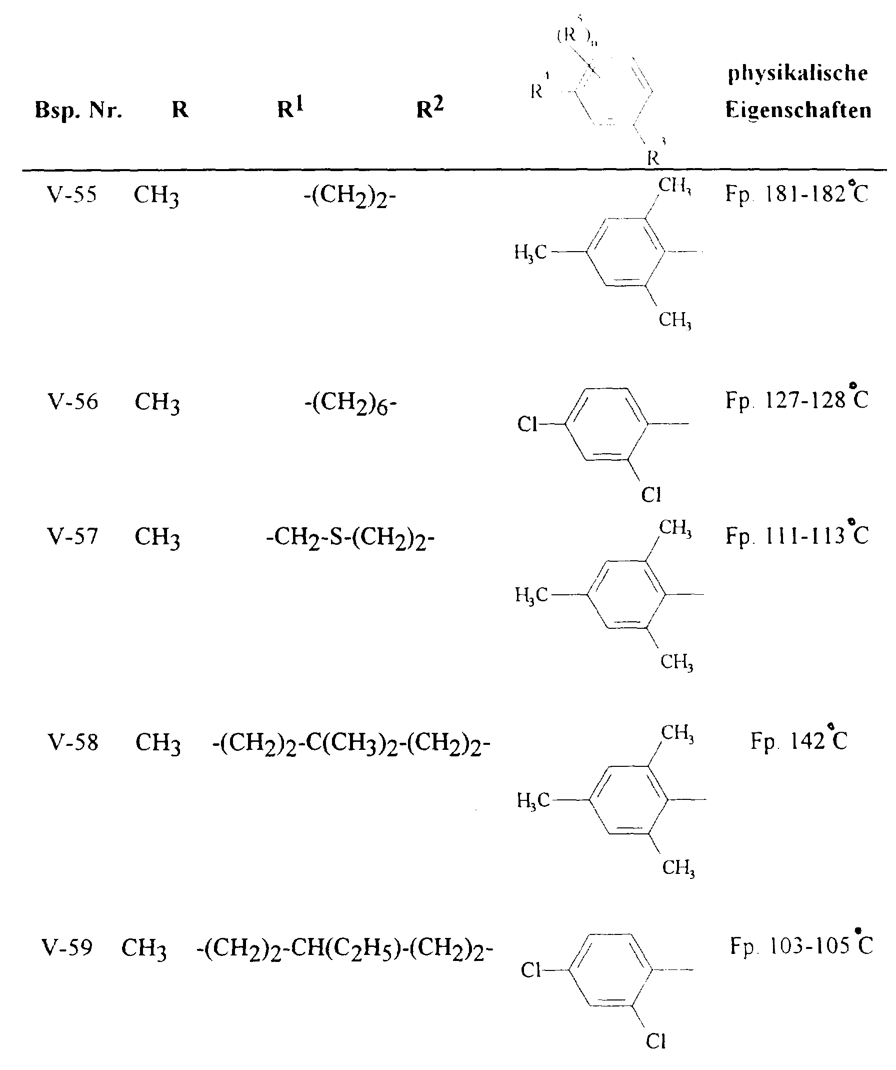

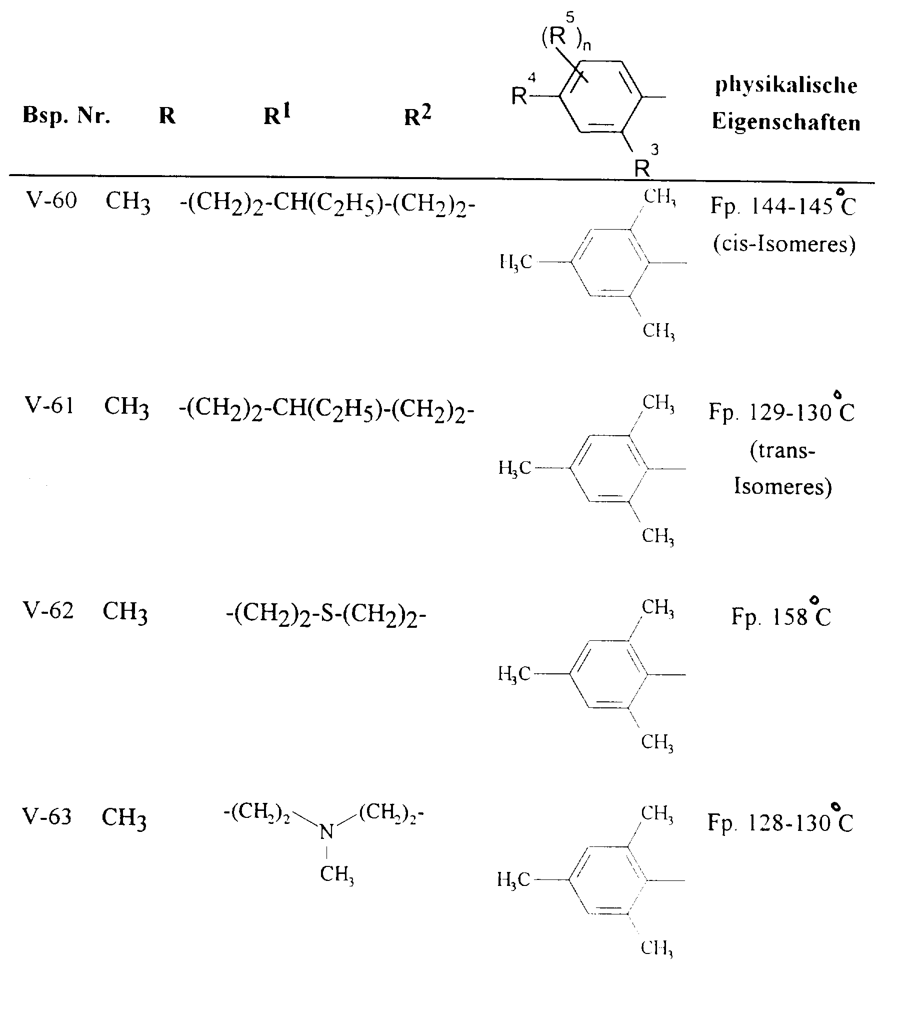

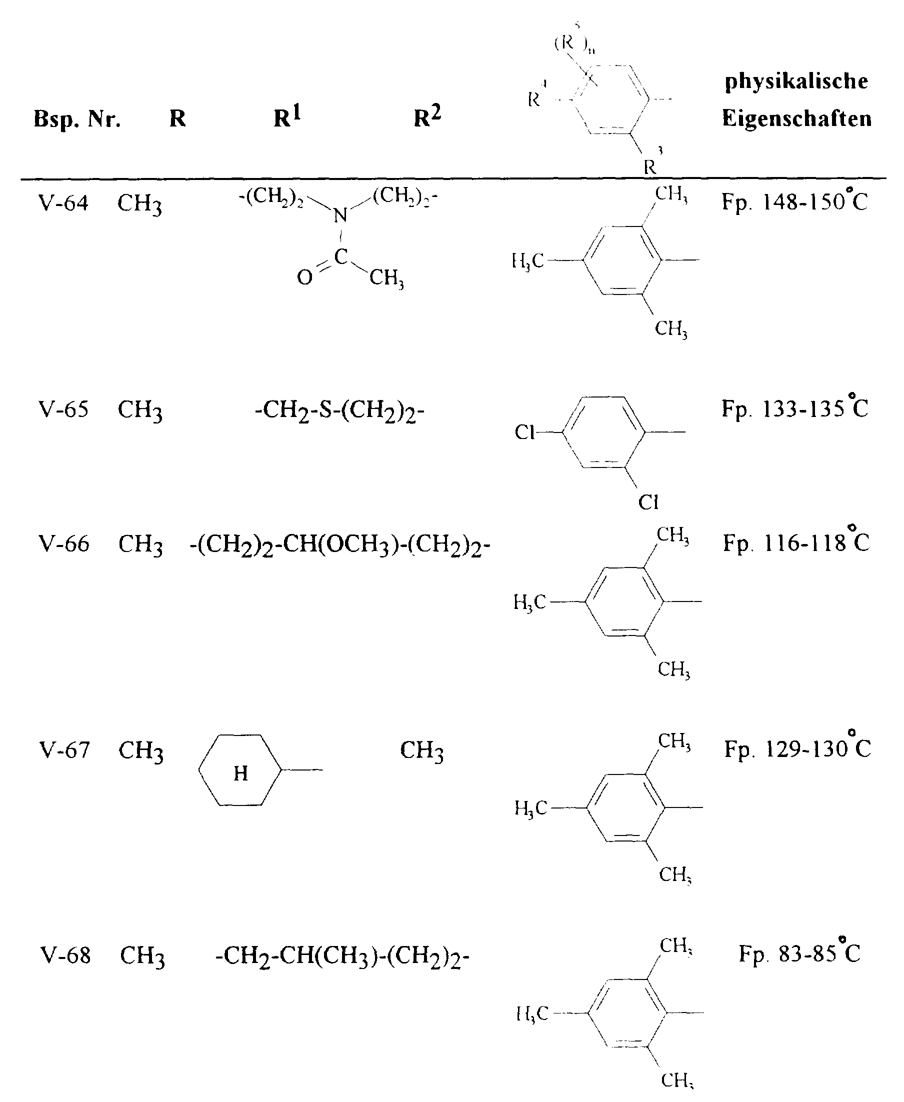

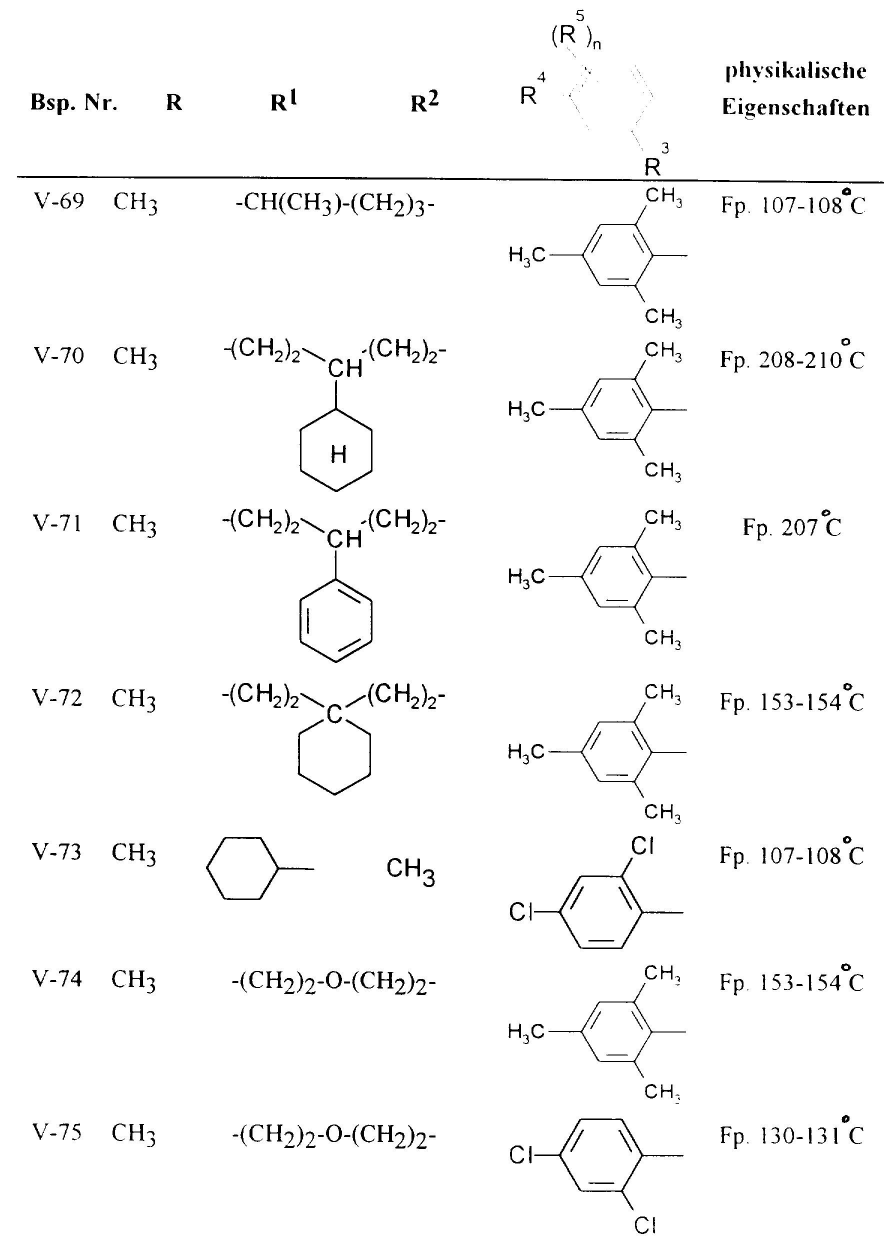

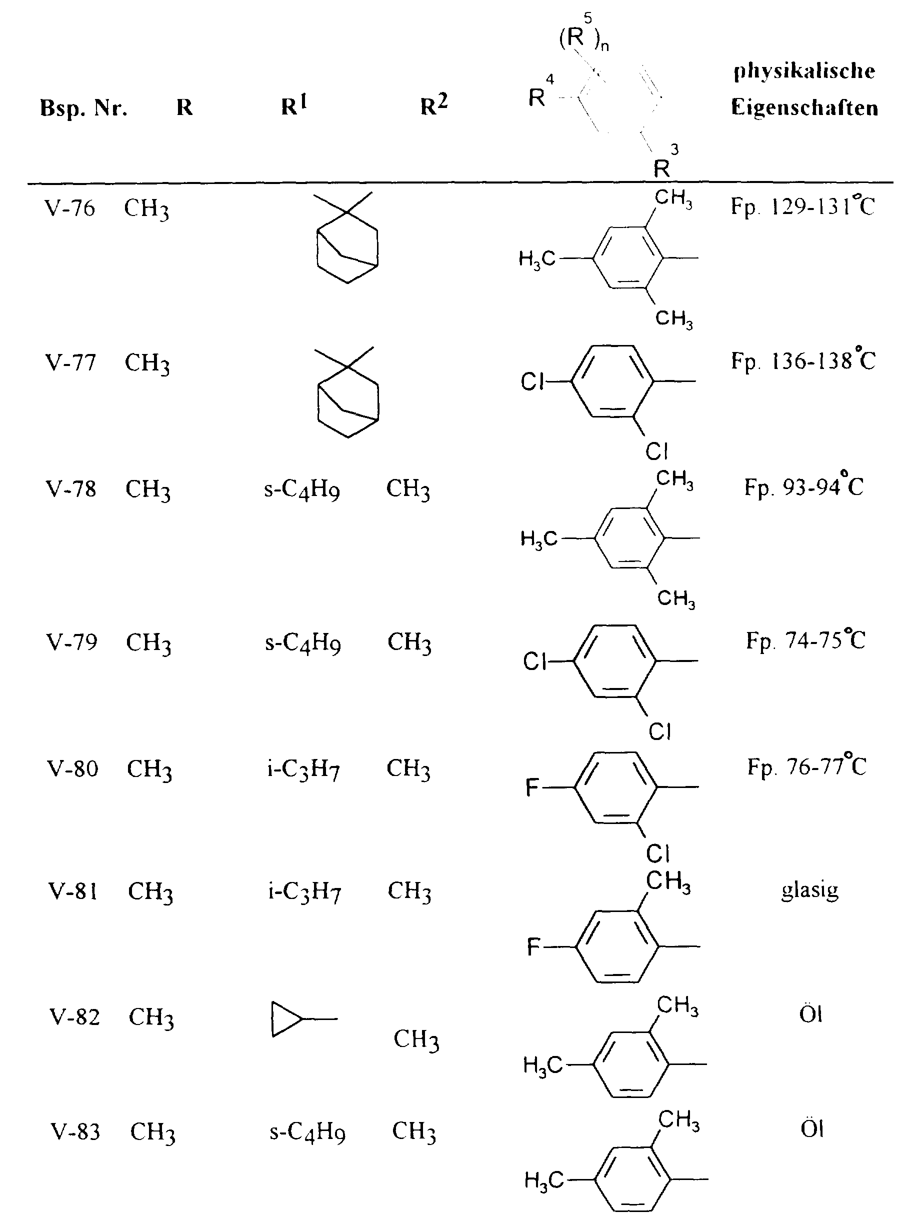

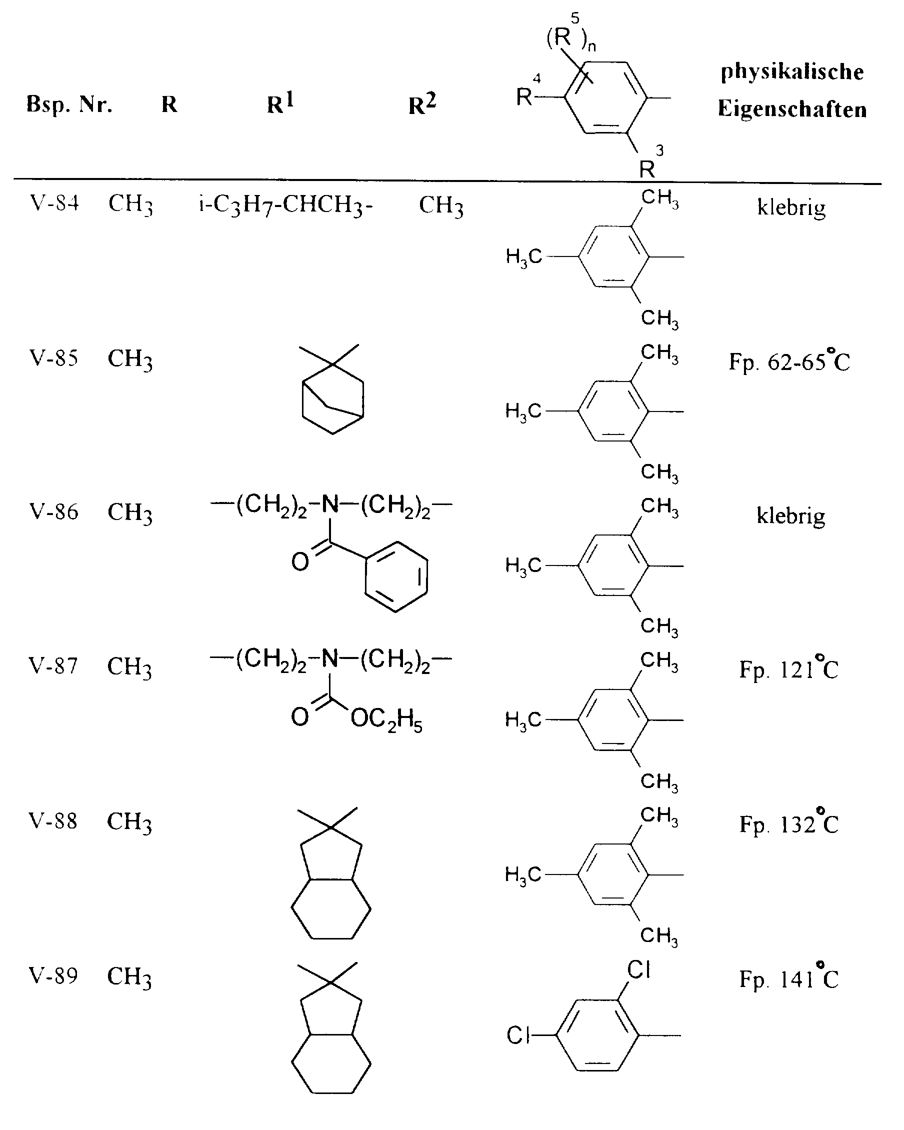

### Beispiel VI-I:

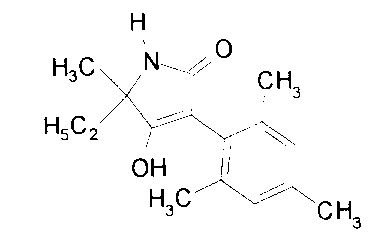

Zu 12,77 g (0,426 Mol) Natriumhydrid in 220 ml absolutem Toluol gibt man bei Rückflußtemperatur tropfenweise 62 g (0,213 Mol) 2-(2,4,6-Trimethylphenyl)-N-(1-methoxycarbonyl-2-butyl)-acetamid in 430 ml Toluol gelöst und kocht die Reaktionsmischung nach beendeter Zugabe so lange bei Rückflußtemperatur bis im Dünnschichtchromatogramm kein Ausgangsprodukt mehr nachweisbar ist. Anschließend gibt man tropfenweise unter Eiskühlung so lange Ethanol zu, bis kein Wasserstoff mehr freigesetzt wird. Zur Aufarbeitung wird im Vakuum eingeengt, der Rückstand in absolutem Ethanol aufgenommen, bei 0°C bis 20°C mit 4N Salzsäure angesäuert, ausgefallener Niederschlag abgesaugt und getrocknet. Das so erhältliche Rohprodukt läßt sich aus Chloroform/n-Hexan (1:2) umkristallisieren

Man erhält 39,4 g (72 % der Theorie) an 5-Ethyl-5-methyl-3-(2,4,6-trimethylphenyl)-pyrrolidin-2,4-dion vom Schmelzpunkt 137-142°C.

## Patentansprüche

1. N-Phenylacetaminonitrile der allgemeinen Formel (I), 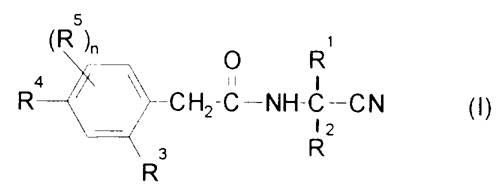 in welcher
R¹ für Wasserstoff oder für jeweils gegebenenfalls substituiertes Alkyl, Alkenyl, Cycloalkyl, Aryl oder Heterocyclyl steht und
R² für Wasserstoff oder für gegebenenfalls substituiertes Alkyl steht oder
R¹ und R² gemeinsam mit dem Kohlenstoffatom, an welches sie gebunden sind, für jeweils gegebenenfalls substituiertes Cycloalkyl oder Heterocyclyl stehen,
R³ für Halogen, Alkyl oder Alkoxy steht,
R⁴ für Wasserstoff, Halogen, Alkyl, Halogenalkyl oder Alkoxy steht,
R⁵ für Halogen, Alkyl oder Alkoxy steht und
n für eine Zahl 0, 1, 2 oder 3 steht.

2. Verfahren zur Herstellung von N-Phenylacetaminonitrilen der allgemeinen Formel (I), 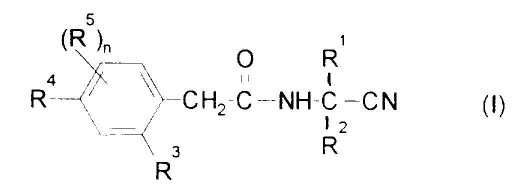 in welcher
R¹ für Wasserstoff oder für jeweils gegebenenfalls substituiertes Alkyl, Alkenyl, Cycloalkyl, Aryl oder Heterocyclyl steht und
R² für Wasserstoff oder für gegebenenfalls substituiertes Alkyl steht oder
R¹ und R² gemeinsam mit dem Kohlenstoffatom, an welches sie gebunden sind, für jeweils gegebenenfalls substituiertes Cycloalkyl oder Heterocyclyl stehen,
R³ für Halogen, Alkyl oder Alkoxy steht,
R⁴ für Wasserstoff Halogen, Alkyl, Halogenalkyl oder Alkoxy steht,
R⁵ für Halogen, Alkyl oder Alkoxy steht und
n für eine Zahl 0, 1, 2 oder 3 steht,
dadurch gekennzeichnet, daß man α-Aminonitrile der Formel (II), 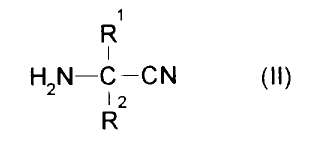 in welcher
R¹ und R² die oben angegebene Bedeutung haben,
mit Phenylessigsäurehalogeniden der Formel (III), 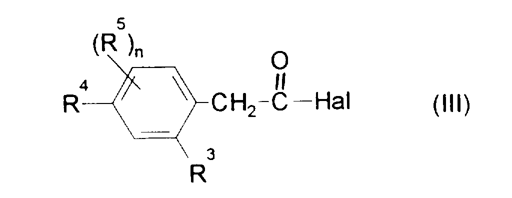 in welcher
R³, R⁴, R⁵ und ndie oben angegebene Bedeutung haben und
Halfür Halogen steht,
gegebenenfalls in Gegenwart eines Verdunnungsmittels und gegebenenfalls in Gegenwart eines Reaktionshilfsmittels umsetzt.

3. Verwendung der N-Phenylacetaminonitrile der Formel (I) gemäß Anspruch 1 als Zwischenprodukte zur Herstellung von 3-Aryl-pyrrolidin-2,4-dionen der Formel (VI), 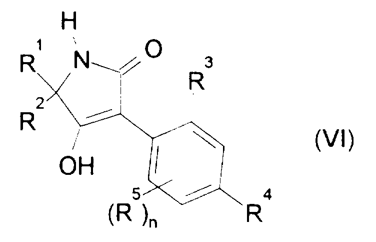 in welcher
R¹, R², R³, R⁴, R⁵ und n die in Anspruch 1 angegebene Bedeutung haben,
indem man sie zunächst in einer ersten Stufe mit Alkoholen der Formel (IV),
R-OH (IV)
in welcher
R für Alkyl steht,
gegebenenfalls in Gegenwart eines Verdünnungsmittels und in Gegenwart von Schwefelsäure als Reaktionshilfsmittels umsetzt und dann die so erhältlichen N-Phenylacetaminocarbonsäureester der Formel (V), 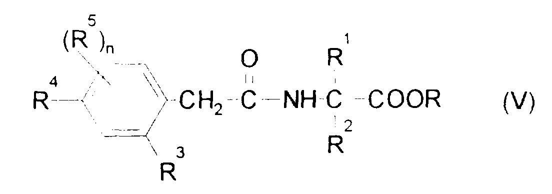 in welcher
R, R¹, R², R³, R⁴, R⁵ und ndie oben angegebene Bedeutung haben,
in einer anschließenden 2.Stufe gegebenenfalls in Gegenwart eines Verdünnungsmittels und gegebenenfalls in Gegenwart eines Reaktionshilfsmittels cyclisiert.

4. N-Phenylacetaminocarbonsäureester der Formel (Va), 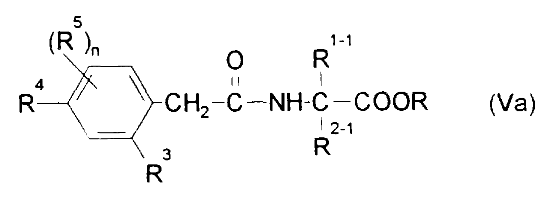 in welcher
R für Alkyl steht,
R¹⁻¹ für Wasserstoff oder für jeweils gegebenenfalls substituiertes Alkyl, Alkenyl, Cycloalkyl, Aryl oder Heterocyclyl steht und
R²⁻¹ für Halogenalkyl steht oder
R¹⁻¹ und R²⁻¹ gemeinsam mit dem Kohlenstoffatom, an welches sie gebunden sind, für jeweils substituiertes Cycloalkyl oder Heterocyclyl stehen und
R³ für Halogen, Alkyl oder Alkoxy steht,
R⁴ für Wasserstoff Halogen, Alkyl, Halogenalkyl oder Alkoxy steht,
R⁵ für Halogen, Alkyl oder Alkoxy steht und
n für eine Zahl 0, 1, 2 oder 3 steht.

5. N-Phenylacetaminonitrile der Formel (I) gemäß Anspruch 1, bei welchen
R¹ für Wasserstoff für jeweils geradkettiges oder verzweigtes Alkyl mit 1 bis 12 Kohlenstoffatomen, Alkenyl mit 3 bis 8 Kohlenstoffatomen, Halogenalkyl mit 1 bis 8 Kohlenstoffatomen und 1 bis 17 gleichen oder verschiedenen Halogenatomen, Alkoxyalkyl mit jeweils 1 bis 8 Kohlenstoffatomen in den einzelnen Alkylteilen, Alkoxyalkoxyalkyl mit jeweils 1 bis 8 Kohlenstoffatomen in den einzelnen Alkylteilen, Alkylthioalkyl mit jeweils 1 bis 8 Kohlenstoffatomen in den einzelnen Alkylteilen, Cycloalkyl mit 3 bis 8 Kohlenstoffatomen, gesättigtes Heterocyclyl mit 3 bis 8 Ringgliedern und 1 bis 2 gleichen oder verschiedenen Heteroatomen - insbesondere Stickstoff, Sauerstoff und/oder Schwefel - oder für jeweils gegebenenfalls im Aryl- bzw. Heteroarylteil einfach oder mehrfach, gleich oder verschieden substituiertes Arylalkyl oder Aryl mit jeweils 6 bis 10 Kohlenstoffatomen im Arylteil und gegebenenfalls 1 bis 6 Kohlenstoffatomen im geradkettigen oder verzweigten Alkylteil oder Heteroaryl mit 2 bis 9 Kohlenstoffatomen und 1 bis 4 gleichen oder verschiedenen Heteroatomen - insbesondere Stickstoff Sauerstoff und/oder Schwefel - steht, wobei als Aryl- bzw. Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen sowie jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen und
R² für Wasserstoff, für jeweils geradkettiges oder verzweigtes Alkyl mit 1 bis 12 Kohlenstoffatomen, Halogenalkyl mit 1 bis 8 Kohlenstoffatomen und 1 bis 17 gleichen oder verschiedenen Halogenatomen oder Alkoxyalkyl mit jeweils 1 bis 8 Kohlenstoffatomen in den einzelnen Alkylteilen steht oder
R¹ und R² gemeinsam mit dem Kohlenstoffatom, an welches sie gebunden sind, für jeweils gegebenenfalls einfach oder mehrfach, gleich oder verschieden substituiertes, gesättigtes oder ungesättigtes Cycloalkyl oder Heterocyclyl mit jeweils 3 bis 12 Kohlenstoffatomen und gegebenenfalls 1 bis 3 gleichen oder verschiedenen Heteroatomen - insbesondere Stickstoff, Sauerstoff und/oder Schwefel - stehen, wobei als Substituenten jeweils infrage kommen:
Halogen, geradkettiges oder verzweigtes Alkyl mit 1 bis 6 Kohlenstoffatomen, geradkettiges oder verzweigtes Alkanoyl mit 1 bis 7 Kohlenstoffatomen, geradkettiges oder verzweigtes Alkandiyl mit 3 bis 8 Kohlenstoffatomen, geradkettiges oder verzweigtes Dioxyalkylen mit 1 bis 8 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxy mit 1 bis 6 Kohlenstoffatomen, geradkettiges oder verzweigtes Alkylthio mit 1 bis 6 Kohlenstoffatomen, Cycloalkyl mit 3 bis 8 Kohlenstoffatomen, Aryl mit 6 bis 10 Kohlenstoffatomen,
R³ für Fluor, Chlor, Brom, Iod, für geradkettiges oder verzweigtes Alkyl mit 1 bis 8 Kohlenstoffatomen oder für geradkettiges oder verzweigtes Alkoxy mit 1 bis 8 Kohlenstoffatomen steht,
R⁴ für Wasserstoff für Fluor, Chlor, Brom, Iod, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für geradkettiges oder verzweigtes Alkoxy mit 1 bis 8 Kohlenstoffatomen steht,
R⁵ für Fluor, Chlor, Brom, Iod, für geradkettiges oder verzweigtes Alkyl mit 1 bis 8 Kohlenstoffatomen oder für geradkettiges oder verzweigtes Alkoxy mit 1 bis 8 Kohlenstoffatomen steht und
n für eine Zahl 0, 1, 2 oder 3 steht

6. Verbindungen der Formel (I) gemaß Anspruch 1, bei welchen
R¹ für Wasserstoff, für jeweils geradkettiges oder verzweigtes Alkyl mit 1 bis 10 Kohlenstoffatomen, Alkenyl mit 3 bis 6 Kohlenstoffatomen, Halogenalkyl mit 1 bis 6 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, Alkoxyalkoxyalkyl mit jeweils 1 bis 6 Kohlenstoffatomen in den einzelnen Alkylteilen, Alkylthioalkyl mit jeweils 1 bis 6 Kohlenstoffatomen in den einzelnen Alkylteilen, Cycloalkyl mit 3 bis 7 Kohlenstoffatomen, gesättigtes Heterocyclyl mit 3 bis 7 Ringgliedern und 1 oder 2 gleichen oder verschiedenen Heteroatomen - insbesondere Stickstoff, Sauerstoff und/oder Schwefel - oder für jeweils gegebenenfalls im Aryl- bzw. Heteroarylteil einfach bis fünffach, gleich oder verschieden substituiertes Arylalkyl oder Aryl mit jeweils 6 oder 10 Kohlenstoffatomen im Arylteil und gegebenenfalls 1 bis 4 Kohlenstoffatomen im geradkettigen oder verzweigten Alkylteil oder Heteroaryl mit 2 bis 9 Kohlenstoffatomen und 1 bis 3 gleichen oder verschiedenen Heteroatomen - insbesondere Stickstoff, Sauerstoff und/oder Schwefel - steht, wobei als Aryl- bzw. Heteroarylsubstituenten jeweils infrage kommen:
Halogen, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen sowie jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen und
R² für Wasserstoff für jeweils geradkettiges oder verzweigtes Alkyl mit 1 bis 10 Kohlenstoffatomen, Halogenalkyl mit 1 bis 6 Kohlenstoffatomen und 1 bis 13 gleichen oder verschiedenen Halogenatomen oder Alkoxyalkyl mit jeweils 1 bis 6 Kohlenstoffatomen in den einzelnen Alkylteilen steht oder
R¹ und R² gemeinsam mit dem Kohlenstoffatom, an welches sie gebunden sind, für jeweils gegebenenfalls einfach bis vierfach, gleich oder verschieden substituiertes, gesättigtes oder ungesättigtes Cycloalkyl oder Heterocyclyl mit jeweils 3 bis 8 Kohlenstoffatomen und gegebenenfalls 1 oder 2 gleichen oder verschiedenen Heteroatomen - insbesondere Stickstoff, Sauerstoff und/oder Schwefel - stehen, wobei als Substituenten jeweils infrage kommen:
Fluor, Chlor, Brom, geradkettiges oder verzweigtes Alkyl mit 1 bis 4 Kohlenstoffatomen, geradkettiges oder verzweigtes Alkanoyl mit 1 bis 5 Kohlenstoffatomen, geradkettiges oder verzweigtes Alkandiyl mit 3 bis 6 Kohlenstoffatomen, geradkettiges oder verzweigtes Dioxyalkylen mit 1 bis 6 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Alkylthio mit 1 bis 4 Kohlenstoffatomen, Cycloalkyl mit 3 bis 7 Kohlenstoffatomen oder Phenyl,
R³ für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht,
R⁴ für Wasserstoff, für Fluor, Chlor, Brom, für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für geradkettiges oder verzweigtes Alkoxy mit 1 bis 6 Kohlenstoffatomen steht,
R⁵ für Fluor, Chlor, Brom, für geradkettiges oder verzweigtes Alkyl mit 1 bis 6 Kohlenstoffatomen oder für geradkettiges oder verzweigtes Alkoxy mit 1 bis 6 Kohlenstoffatomen steht und
n für eine Zahl 0, 1 oder 2 steht.

7. Verbindungen der Formel (I) gemäß Anspruch 1, bei welchen
R¹ für Wasserstoff, für jeweils geradkettiges oder verzweigtes Alkyl mit 1 bis 8 Kohlenstoffatomen, Alkenyl mit 3 bis 5 Kohlenstoffatomen, Halogenalkyl mit 1 bis 4 Kohlenstoffatomen und 1 bis 9 gleichen oder verschiedenen Halogenatomen, Alkoxyalkyl mit jeweils 1 bis 3 Kohlenstoffatomen in den einzelnen Alkylteilen, Alkoxyalkoxyalkyl mit jeweils 1 bis 3 Kohlenstoffatomen in den einzelnen Alkylteilen, Alkylthioalkyl mit jeweils 1 bis 3 Kohlenstoffatomen in den einzelnen Alkylteilen, Cycloalkyl mit 3 bis 6 Kohlenstoffatomen, gesättigtes Heterocyclyl mit 3, 5 oder 6 Ringgliedern und 1 Heteroatom - insbesondere Stickstoff Sauerstoff oder Schwefel - oder für jeweils gegebenenfalls im Phenyl- bzw. Heteroarylteil einfach bis dreifach, gleich oder verschieden substituiertes Phenylalkyl oder Phenyl mit gegebenenfalls 1 bis 3 Kohlenstoffatomen im Alkylteil oder Heteroaryl - insbesondere Pyridyl, Imidazolyl, Pyrazolyl, Triazolyl oder Thiazolyl - steht, wobei als Phenyl- bzw. Heteroarylsubstituenten jeweils infrage kommen:
Fluor, Chlor, Brom, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
R² für Wasserstoff, für jeweils geradkettiges oder verzweigtes Alkyl mit 1 bis 6 Kohlenstoffatomen, Halogenalkyl mit 1 bis 4 Kohlenstoffatomen und 1 bis 9 gleichen oder verschiedenen Halogenatomen oder Alkoxyalkyl mit jeweils 1 bis 4 Kohlenstoffatomen in den einzelnen Alkylteilen steht oder
R¹ und R² gemeinsam mit dem Kohlenstoffatom, an welches sie gebunden sind, für gegebenenfalls einfach bis dreifach, gleich oder verschieden substituiertes, gesättigtes oder ungesättigtes Cycloalkyl oder Heterocyclyl jeweils mit 3, 5, 6, 7 oder 8 Kohlenstoffatomen und gegebenenfalls 1 oder 2 gleichen oder verschiedenen Heteroatomen - insbesondere Stickstoff, Sauerstoff und/oder Schwefel - stehen, wobei als Substituenten jeweils infrage kommen:
Fluor, Chlor, Brom, geradkettiges oder verzweigtes Alkyl mit 1 bis 4 Kohlenstoffatomen, geradkettiges oder verzweigtes Alkanoyl mit 1 bis 4 Kohlenstoffatomen, Dioxyalkylen mit 1 bis 3 Kohlenstoffatomen, Halogenalkyl mit 1 oder 2 Kohlenstoffatomen und 1 bis 5 gleichen oder verschiedenen Halogenatomen, geradkettiges oder verzweigtes Alkoxy mit 1 oder 2 Kohlenstoffatomen, geradkettiges oder verzweigtes Alkylthio mit 1 oder 2 Kohlenstoffatomen, Cycloalkyl mit 3, 5 oder 6 Kohlenstoffatomen oder Phenyl,
R³ für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht,
R⁴ für Wasserstoff, für Fluor, Chlor, Brom, für geradkettiges oder verzweigtes Alkyl mit 1 bis 4 Kohlenstoffatomen, für Halogenalkyl mit 1 oder 2 Kohlenstoffatomen und 1 bis 5 gleichen oder verschiedenen Halogenatomen oder für geradkettiges oder verzweigtes Alkoxy mit 1 bis 3 Kohlenstoffatomen steht,
R⁵ für Fluor, Chlor, Brom, für geradkettiges oder verzweigtes Alkyl mit 1 bis 4 Kohlenstoffatomen oder für geradkettiges oder verzweigtes Alkoxy mit 1 bis 3 Kohlenstoffatomen steht und
n für eine Zahl 0 oder 1 steht.

## Claims

1. N-Phenylacetaminonitriles of the general formula (I), 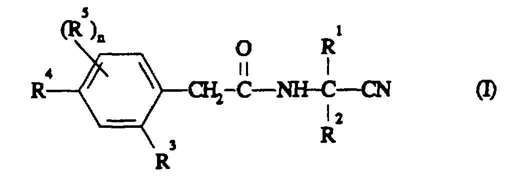 in which
R¹ represents hydrogen, or represents alkyl, alkenyl, cycloalkyl, aryl or heterocyclyl which are in each case optionally substituted, and
R² represents hydrogen, or represents alkyl which is optionally substituted, or
R¹ and R², together with the carbon atom to which they are bonded, represent cycloalkyl or heterocyclyl which are in each case optionally substituted,
R³ represents halogen, alkyl or alkoxy,
R⁴ represents hydrogen, halogen, alkyl, halogenoalkyl or alkoxy,
R⁵ represents halogen, alkyl or alkoxy, and
n represents a number 0, 1, 2 or 3.

2. Process for preparing N-phenylacetaminonitriles of the general formula (I), 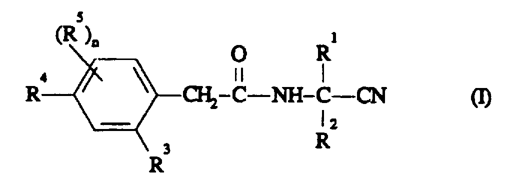 in which
R¹ represents hydrogen, or represents alkyl, alkenyl, cycloalkyl, aryl or heterocyclyl which are in each case optionally substituted, and
R² represents hydrogen, or represents alkyl which is optionally substituted, or
R¹ and R², together with the carbon atom to which they are bonded, represent cycloalkyl or heterocyclyl which are in each case optionally substituted,
R³ represents halogen, alkyl or alkoxy,
R⁴ represents hydrogen, halogen, alkyl, halogenoalkyl or alkoxy,
R⁵ represents halogen, alkyl or alkoxy, and
n represents a number 0, 1, 2 or 3,
characterised in that α-aminonitriles of the formula (II), 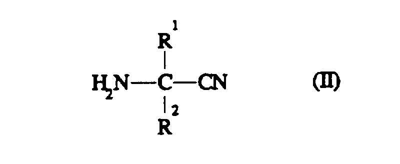 in which
R¹ and R² have the abovementioned meaning,
are reacted with phenylacetyl halides of the formula (III), 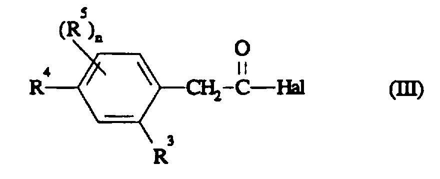 in which
R³, R⁴, R⁵ and n have the abovementioned meaning, and
Hal represents halogen,
optionally in the presence of a diluent and optionally in the presence of a reaction aid.

3. Use of the N-phenylacetaminonitriles of the formula (I) according to Claim 1 as intermediates for the preparation of 3-aryl-pyrrolidine-2,4-diones of the formula (VI), 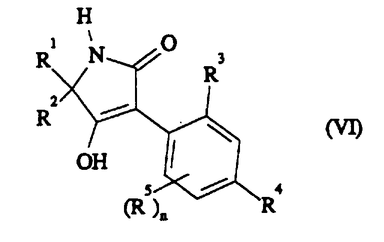 in which
R¹, R², R³, R⁴, R⁵ and n have the meaning given in Claim 1,
by initially reacting them in a first stage with alcohols of the formula (IV),
R-OH (IV)
in which
R represents alkyl,
optionally in the presence of a diluent and in the presence of sulphuric acid as a reaction aid, and then cyclising the N-phenylacetaminocarboxylic acid esters of the formula (V) which can thus be obtained, 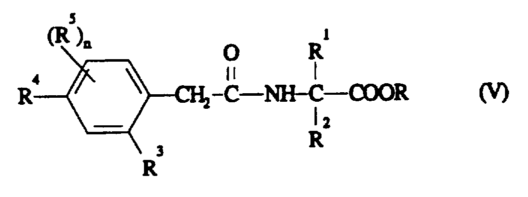 in which
R, R¹, R², R³, R⁴, R⁵ and n have the abovementioned meaning,
in a subsequent second stage, optionally in the presence of a diluent and optionally in the presence of a reaction aid.

4. N-Phenylacetaminocarboxylic acid esters of the formula (Va), 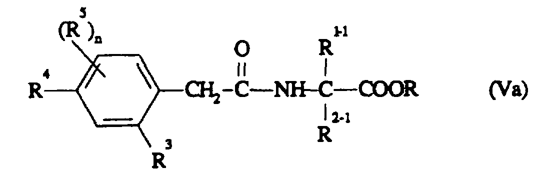 in which
R represents alkyl,
R¹⁻¹ represents hydrogen, or represents alkyl, alkenyl, cycloalkyl, aryl or heterocyclyl, which are in each case optionally substituted, and
R²⁻¹ represents halogenoalkyl, or
R¹⁻¹ and R²⁻¹, together with the carbon atom to which they are bonded, represent cycloalkyl or heterocyclyl which are in each case substituted, and
R³ represents halogen, alkyl or alkoxy,
R⁴ represents hydrogen, halogen, alkyl, halogenoalkyl or alkoxy,
R⁵ represents halogen, alkyl or alkoxy, and
n represents a number 0, 1, 2 or 3.

5. N-Phenylacetaminonitriles of the formula (I) according to Claim 1, in which
R¹ represents hydrogen, in each case straight-chain or branched alkyl having 1 to 12 carbon atoms, alkenyl having 3 to 8 carbon atoms, halogenoalkyl having 1 to 8 carbon atoms and 1 and 17 identical or different halogen atoms, alkoxyalkyl having in each case 1 to 8 carbon atoms in the individual alkyl moieties, alkoxyalkoxyalkyl having in each case 1 to 8 carbon atoms in the individual alkyl moieties, alkylthioalkyl having in each case 1 to 8 carbon atoms in the individual alkyl moieties, cycloalkyl having 3 to 8 carbon atoms, saturated heterocyclyl having 3 to 8 ring members and 1 to 2 identical or different heteroatoms - in particular nitrogen, oxygen and/or sulphur - or represents arylalkyl or aryl, having in each case 6 to 10 carbon atoms in the aryl moiety and, where appropriate, 1 to 6 carbon atoms in the straight-chain or branched alkyl moiety, or heteroaryl, having 2 to 9 carbon atoms and 1 to 4 identical or different heteroatoms - in particular nitrogen, oxygen and/or sulphur, which are in each case optionally substituted identically or differently once or more than once in the aryl or heteroaryl moiety, where suitable aryl or heteroaryl substituents in each case are :
halogen, nitro, in each case straight-chain or branched alkyl, alkoxy, alkylthio, alkylsulphinyl or alkylsulphonyl having in each case 1 to 6 carbon atoms, as well as in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, and
R² represents hydrogen, in each case straight-chain or branched alkyl having 1 to 12 carbon atoms, halogenoalkyl having 1 to 8 carbon atoms and 1 to 17 identical or different halogen atoms, or alkoxyalkyl having in each case 1 to 8 carbon atoms in the individual alkyl moieties, or
R¹ and R², together with the carbon atom to which they are bonded, represent saturated or unsaturated cycloalkyl or heterocyclyl having in each case 3 to 12 carbon atoms and, where appropriate, 1 to 3 identical or different heteroatoms - in particular nitrogen, oxygen and/or sulphur, which are in each case optionally substituted identically or differently once or more than once, where suitable substituents in each case are :
halogen, straight-chain or branched alkyl having 1 to 6 carbon atoms, straight-chain or branched alkanoyl having 1 to 7 carbon atoms, straight-chain or branched alkanediyl having 3 to 8 carbon atoms, straight-chain or branched dioxyalkylene having 1 to 8 carbon atoms, straight-chain or branched halogenoalkyl having 1 to 6 carbon atoms and 1 to 13 identical or different halogen atoms, straight-chain or branched alkoxy having 1 to 6 carbon atoms, straight-chain or branched alkylthio having 1 to 6 carbon atoms, cycloalkyl having 3 to 8 carbon atoms or aryl having 6 to 10 carbon atoms,
R³ represents fluorine, chlorine, bromine, iodine, straight-chain or branched alkyl having 1 to 8 carbon atoms, or straight-chain branched alkoxy having 1 to 8 carbon atoms,
R⁴ represents hydrogen, fluorine, chlorine, bromine, iodine, straight-chain or branched alkyl having 1 to 8 carbon atoms, straight-chain or branched halogenoalkyl having 1 to 8 carbon atoms and 1 to 17 identical or different halogen atoms, or straight-chain or branched alkoxy having 1 to 8 carbon atoms,
R⁵ represents fluorine, chlorine, bromine, iodine, straight-chain or branched alkyl having 1 to 8 carbon atoms, or straight-chain or branched alkoxy having 1 to 8 carbon atoms, and
n represents a number 0, 1, 2 or 3.

6. Compounds of the formula (I) according to Claim 1, in which
R¹ represents hydrogen, in each case straight-chain or branched alkyl having 1 to 10 carbon atoms, alkenyl having 3 to 6 carbon atoms, halogenoalkyl having 1 to 6 carbon atoms and 1 to 13 identical or different halogen atoms, alkoxyalkyl having in each case 1 to 6 carbon atoms in the individual alkyl moieties, alkoxyalkoxyalkyl having in each case 1 to 6 carbon atoms in the individual alkyl moieties, alkylthioalkyl having in each case 1 to 6 carbon atoms in the individual alkyl moieties, cycloalkyl having 3 to 7 carbon atoms, saturated heterocyclyl having 3 to 7 ring members and 1 or 2 identical or different heteroatoms - in particular nitrogen, oxygen and/or sulphur - or represents arylalkyl or aryl, having in each case 6 or 10 carbon atoms in the aryl moiety and, where appropriate, 1 to 4 carbon atoms in the straight-chain or branched alkyl moiety, or heteroaryl, having 2 to 9 carbon atoms and 1 to 3 identical or different heteroatoms - in particular nitrogen, oxygen and/or sulphur, which are in each case optionally substituted identically or differently once to five times in the aryl or heteroaryl moiety, where suitable aryl or heteroaryl substituents in each case are :
halogen, nitro, in each case straight-chain or branched alkyl, alkoxy, alkylthio, alkylsulphinyl or alkylsulphonyl having in each case 1 to 4 carbon atoms, as well as in each case straight-chain or branched halogenoalkyl, halogenoalkoxy, halogenoalkylthio, halogenoalkylsulphinyl or halogenoalkylsulphonyl having in each case 1 to 4 carbon atoms and 1 to 9 identical or different halogen atoms, and
R² represents hydrogen, straight-chain or branched alkyl having 1 to 10 carbon atoms, halogenoalkyl having 1 to 6 carbon atoms and 1 to 13 identical or different halogen atoms, or alkoxyalkyl having in each case 1 to 6 carbon atoms in the individual alkyl moieties, or
R¹ and R², together with the carbon atom to which they are bonded, represent saturated or unsaturated cycloalkyl or heterocyclyl having in each case 3 to 8 carbon atoms and, where appropriate, or 1 or 2 identical or different heteroatoms - in particular nitrogen, oxygen and/or sulphur, which are in each case optionally substituted identically or differently once to four times, where suitable substituents in each case are:
fluorine, chlorine, bromine, straight-chain or branched alkyl having 1 to 4 carbon atoms, straight-chain or branched alkanoyl having 1 to 5 carbon atoms, straight-chain or branched alkanediyl having 3 to 6 carbon atoms, straight-chain or branched dioxyalkylene having 1 to 6 carbon atoms, straight-chain or branched halogenoalkyl having 1 to 4 carbon atoms and 1 to 9 identical or different halogen atoms, straight-chain or branched alkoxy having 1 to 4 carbon atoms, straight-chain or branched alkylthio having 1 to 4 carbon atoms, cycloalkyl having 3 to 7 carbon atoms, or phenyl,
R³ represents fluorine, chlorine, bromine, straight-chain or branched alkyl having 1 to 6 carbon atoms, or straight-chain or branched alkoxy having 1 to 6 carbon atoms,
R⁴ represents hydrogen, fluorine, chlorine, bromine, straight-chain or branched alkyl having 1 to 6 carbon atoms, straight-chain or branched halogenoalkyl having 1 to 6 carbon atoms and 1 to 13 identical or different halogen atoms, or straight-chain or branched alkoxy having 1 to 6 carbon atoms,
R⁵ represents fluorine, chlorine, bromine, straight-chain or branched alkyl having 1 to 6 carbon atoms, or straight-chain or branched alkoxy having 1 to 6 carbon atoms, and
n represents a number 0, 1 or 2.

7. Compounds of the formula (I) according to Claim 1, in which
R¹ represents hydrogen, in each case straight-chain or branched alkyl having 1 to 8 carbon atoms, alkenyl having 3 to 5 carbon atoms, halogenoalkyl having 1 to 4 carbon atoms and 1 to 9 identical or different halogen atoms, alkoxyalkyl having in each case 1 to 3 carbon atoms in the individual alkyl moieties, alkoxyalkoxyalkyl having in each case 1 to 3 carbon atoms in the individual alkyl moieties, alkylthioalkyl having in each case 1 to 3 carbon atoms in the individual alkyl moieties, cycloalkyl having 3 to 6 carbon atoms, saturated heterocyclyl having 3, 5 or 6 ring members and 1 heteroatom - in particular nitrogen, oxygen or sulphur - or represents phenylalkyl or phenyl, having optionally 1 to 3 carbon atoms in the alkyl moiety, or heteroaryl - in particular pyridyl, imidazolyl, pyrazolyl, triazolyl or thiazolyl, which are in each case optionally substituted identically or differently once to three times in the phenyl or heteroaryl moiety, where suitable phenyl or heteroaryl substituents in each case are:
fluorine, chlorine, bromine, nitro, methyl, ethyl, n- or i-propyl, n-, i-, s- or t-butyl, methoxy, ethoxy, n-, or i-propoxy, n-, i-, s- or t-butoxy, methylthio, ethylthio, methylsulphinyl, methylsulphonyl, trifluoromethyl, difluoromethyl, trifluoromethoxy, difluoromethoxy, trifluoromethylthio, trifluoromethylsulphinyl or trifluoromethylsulphonyl,
R² represents hydrogen, in each case straight-chain or branched alkyl having 1 to 6 carbon atoms, halogenoalkyl having 1 to 4 carbon atoms and 1 to 9 identical or different halogen atoms, or alkoxyalkyl having in each case 1 to 4 carbon atoms in the individual alkyl moieties, or
R¹ and R², together with the carbon atom to which they are bonded, represent saturated or unsaturated cycloalkyl or heterocyclyl having in each case 3, 5, 6, 7 or 8 carbon atoms and, where appropriate, 1 or 2 identical or different heteroatoms - in particular nitrogen, oxygen and/or sulphur, which are optionally substituted identically or differently once to three times, where in each case suitable substituents are:
fluorine, chlorine, bromine, straight-chain or branched alkyl having 1 to 4 carbon atoms, straight-chain or branched alkanoyl having 1 to 4 carbon atoms, dioxyalkylene having 1 to 3 carbon atoms, halogenoalkyl having 1 or 2 carbon atoms and 1 to 5 identical or different halogen atoms, straight-chain or branched alkoxy having 1 or 2 carbon atoms, straight-chain or branched alkylthio having 1 or 2 carbon atoms, cycloalkyl having 3, 5 or 6 carbon atoms, or phenyl,
R³ represents fluorine, chlorine, bromine, straight-chain or branched alkyl having 1 to 4 carbon atoms, or straight-chain or branched alkoxy having 1 to 3 carbon atoms,
R⁴ represents hydrogen, fluorine, chlorine, bromine, straight-chain or branched alkyl having 1 to 4 carbon atoms, halogenoalkyl having 1 or 2 carbon atoms and 1 to 5 identical or different halogen atoms, or straight-chain or branched alkoxy having 1 to 3 carbon atoms,
R⁵ represents fluorine, chlorine, bromine, straight-chain or branched alkyl having 1 to 4 carbon atoms, or straight-chain or branched alkoxy having 1 to 3 carbon atoms, and
n represents a number 0 or 1.

## Revendications

1. N-phénylacétaminonitriles répondant à la formule générale (I) 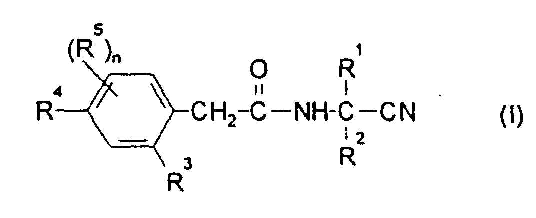 dans laquelle
R¹ représente un atome d'hydrogène; ou un groupe alkyle, un groupe alcényle, un groupe cycloalkyle, un groupe aryle ou un groupe hétérocyclyle, chacun de ces groupes portant éventuellement un ou plusieurs substituants identiques ou différents, et
R² représente un atome d'hydrogène ou un groupe alkyle portant éventuellement un ou plusieurs substituants identiques ou différents, ou bien
R¹ et R² représentent, ensemble avec l'atome de carbone auquel ils sont liés, un groupe cycloalkyle ou hétérocyclyle, chacun de ces groupes portant éventuellement un ou plusieurs substituants identiques ou différents,
R³ représente un atome d'halogène, un groupe alkyle ou un groupe alcoxy,
R⁴ représente un atome d'hydrogène, un atome d'halogène, un groupe alkyle, un groupe halogénalkyle ou un groupe alcoxy,
R⁵ représente un atome d'halogène, un groupe alkyle ou un groupe alcoxy, et
n représente le nombre 0, 1, 2 ou 3.

2. Procédé pour la préparation de N-phénylacétaminonitriles répondant à la formule générale (I) 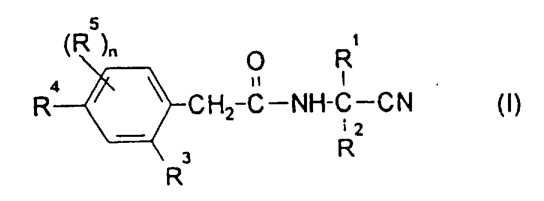 dans laquelle
R¹ représente un atome d'hydrogène; ou un groupe alkyle, un groupe alcényle, un groupe cycloalkyle, un groupe aryle ou un groupe hétérocyclyle, chacun de ces groupes portant éventuellement un ou plusieurs substituants identiques ou différents, et
R² représente un atome d'hydrogène ou un groupe alkyle portant éventuellement un ou plusieurs substituants identiques ou différents, ou bien
R¹ et R² représentent, ensemble avec l'atome de carbone auquel ils sont liés, un groupe cycloalkyle ou hétérocyclyle, chacun de ces groupes portant éventuellement un ou plusieurs substituants identiques ou différents,
R³ représente un atome d'halogène, un groupe alkyle ou un groupe alcoxy,
R⁴ représente un atome d'hydrogène, un atome d'halogène, un groupe alkyle, un groupe halogénalkyle ou un groupe alcoxy,
R⁵ représente un atome d'halogène, un groupe alkyle ou un groupe alcoxy, et
n représente le nombre 0, 1, 2 ou 3,
caractérisé en ce qu'on fait réagir des α-aminonitriles de formule (II) 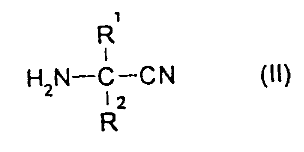 dans laquelle
R¹ et R² ont la signification indiquée ci-dessus,
avec des halogénures d'acide phénylacétique de formule (III) 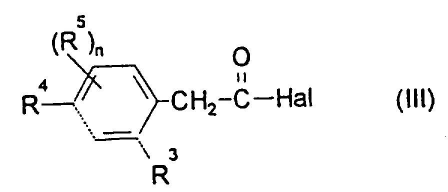 dans laquelle
R³, R⁴, R⁵ et n ont la signification indiquée ci-dessus, et Hal représente un atome d'halogène,
éventuellement en présence d'un diluant et éventuellement en présence d'un adjuvant réactionnel.

3. Utilisation des N-phénylacétaminonitriles de formule (I) selon la revendication 1, comme produits intermédiaires pour la préparation de 3-aryl-pyrrolidin-2,4-diones de formule (VI) 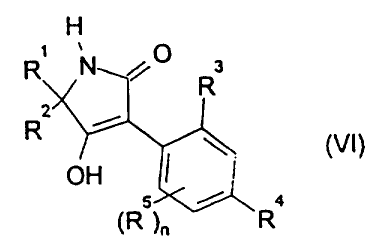 dans laquelle
R¹, R², R³, R⁴, R⁵ et n ont la signification indiquée à la revendication 1,
en les faisant réagir d'abord dans une première étape avec des alcools de formule (IV)
R-OH (IV)
dans laquelle
R représente un groupe alkyle,
éventuellement en présence d'un diluant et en présence d'acide sulfurique, comme adjuvant réactionnel, et ensuite, on cyclise les esters N-phénylacétamino-carboxyliques obtenus de formule (V) 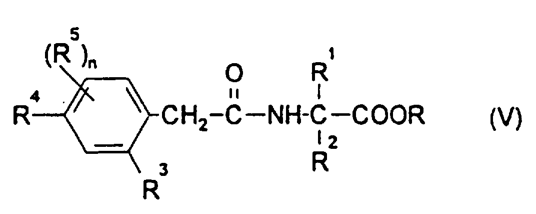 dans laquelle
R, R¹, R², R³, R⁴, R⁵ et n ont la signification indiquée ci-dessus,
dans une seconde étape ultérieure, éventuellement en présence d'un diluant et éventuellement en présence d'un adjuvant réactionnel.

4. Esters N-phénylacétaminocarboxyliques de formule (Va) 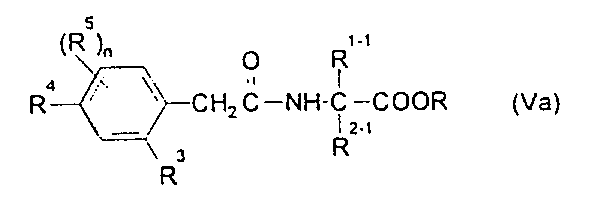 dans laquelle
R représente un groupe alkyle,
R¹⁻¹ représente un atome d'hydrogène ou un groupe alkyle, un groupe alcényle, un groupe cycloalkyle, un groupe aryle ou un groupe hétérocyclyle, chacun de ces groupes portant éventuellement un ou plusieurs substituants identiques ou différents, et
R²⁻¹ représente un groupe halogénalkyle, ou bien
R¹⁻¹ et R²⁻¹ représentent, ensemble avec l'atome de carbone auquel ils sont liés, un groupe cycloalkyle ou un radical hétérocyclyle, chacun de ces groupes étant substitué de manière identique ou différente,
R³ représente un atome d'halogène, un groupe alkyle ou un groupe alcoxy,
R⁴ représente un atome d'hydrogène, un atome d'halogène, un groupe alkyle, un groupe halogénalkyle ou un groupe alcoxy,
R⁵ représente un atome d'halogène, un groupe alkyle ou un groupe alcoxy, et
n représente le chiffre 0, 1, 2 ou 3.

5. N-phénylacétaminonitriles de formule (I) selon la revendication 1, dans lesquels
R¹ représente un atome d'hydrogène; un groupe alkyle contenant de 1 à 12 atomes de carbone, un groupe alcényle contenant de 3 à 8 atomes de carbone, un groupe halogénalkyle contenant de 1 à 8 atomes de carbone et de 1 à 17 atomes d'halogène identiques ou différents, un groupe alcoxyalkyle contenant respectivement de 1 à 8 atomes de carbone dans les fractions alkyle individuelles, un groupe alcoxyalcoxyalkyle contenant respectivement de 1 à 8 atomes de carbone dans les fractions alkyle individuelles, un groupe alkylthioalkyle contenant respectivement de 1 à 8 atomes de carbone dans les fractions alkyle individuelles, chacun de ces groupes étant à chaîne droite ou ramifiée; un groupe cycloalkyle contenant de 3 à 8 atomes de carbone, un groupe hétérocyclyle saturé contenant de 3 à 8 chaînons et de 1 à 2 hétéroatomes identiques ou différents - en particulier un atome d'azote, un atome d'oxygène et/ou un atome de soufre -; ou un groupe arylalkyle ou un groupe aryle contenant respectivement de 6 à 10 atomes de carbone dans la fraction aryle et éventuellement de 1 à 6 atomes de carbone dans la fraction alkyle à chaîne droite ou ramifiée, ou encore un groupe hétéroaryle contenant de 2 à 9 atomes de carbone et de 1 à 4 hétéroatomes identiques ou différents - en particulier un atome d'azote, un atome d'oxygène et/ou un atome de soufre -, chacun de ces groupes portant éventuellement, dans la fraction aryle, respectivement hétéroaryle, un ou plusieurs substituants identiques ou différents, dans lesquels, comme substituants du groupe aryle, respectivement du groupe hétéroaryle, on envisage respectivement:
un atome d'halogène, un groupe nitro; un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée; ainsi qu'un groupe halogénalkyle, un groupe halogénalcoxy, un groupe halogénalkylthio, un groupe halogénalkylsulfinyle ou un groupe halogénalkylsulfonyle contenant respectivement de 1 à 6 atomes de carbone et de 1 à 13 atomes d'halogène identiques ou différents, chacun de ces groupes étant à chaîne droite ou ramifiée, et
R² représente un atome d'hydrogène; un groupe alkyle contenant de 1 à 12 atomes de carbone, un groupe halogénalkyle contenant de 1 à 8 atomes de carbone et de 1 à 17 atomes d'halogène identiques ou différents, ou encore un groupe alcoxyalkyle contenant respectivement de 1 à 8 atomes de carbone dans les fractions alkyle individuelles, chacun de ces groupes étant à chaîne droite ou ramifiée, ou bien
R¹ et R² représentent, ensemble avec l'atome de carbone auquel ils sont liés, un groupe cycloalkyle ou un groupe hétérocyclyle saturé ou insaturé, contenant respectivement de 3 à 12 atomes de carbone et éventuellement de 1 à 3 hétéroatomes identiques ou différents - en particulier un atome d'azote, un atome d'oxygène et/ou un atome de soufre -, chacun de ces groupes portant éventuellement un ou plusieurs substituants identiques ou différents, dans lesquels, comme substituants, on envisage respectivement:
un atome d'halogène, un groupe alkyle à chaîne droite ou ramifiée contenant de 1 à 6 atomes de carbone, un groupe alcanoyle à chaîne droite ou ramifiée contenant de 1 à 7 atomes de carbone, un groupe alcanediyle à chaîne droite ou ramifiée contenant de 3 à 8 atomes de carbone, un radical dioxyalkylène à chaîne droite ou ramifiée contenant de 1 à 8 atomes de carbone, un groupe halogénalkyle à chaîne droite ou ramifiée contenant de 1 à 6 atomes de carbone et de 1 à 13 atomes d'halogène identiques ou différents, un groupe alcoxy à chaîne droite ou ramifiée contenant de 1 à 6 atomes de carbone, un groupe alkylthio à chaîne droite ou ramifiée contenant de 1 à 6 atomes de carbone, un groupe cycloalkyle contenant de 3 à 8 atomes de carbone, un groupe aryle contenant de 6 à 10 atomes de carbone,
R³ représente un atome de fluor, un atome de chlore, un atome de brome, un atome d'iode, un groupe alkyle à chaîne droite ou ramifiée contenant de 1 à 8 atomes de carbone ou un groupe alcoxy à chaîne droite ou ramifiée contenant de 1 à 8 atomes de carbone,
R⁴ représente un atome d'hydrogène, un atome de fluor, un atome de chlore, un atome de brome, un atome d'iode, un groupe alkyle à chaîne droite ou ramifiée contenant de 1 à 8 atomes de carbone, un groupe halogénalkyle à chaîne droite ou ramifiée contenant de 1 à 8 atomes de carbone et de 1 à 17 atomes d'halogène identiques ou différents, ou encore un groupe alcoxy à chaîne droite ou ramifiée contenant de 1 à 8 atomes de carbone,
R⁵ représente un atome de fluor, un atome de chlore, un atome de brome, un atome d'iode, un groupe alkyle à chaîne droite ou ramifiée contenant de 1 à 8 atomes de carbone ou un groupe alcoxy à chaîne droite ou ramifiée contenant de 1 à 8 atomes de carbone, et
n représente le chiffre 0, 1, 2 ou 3.

6. Composés de formule (I) selon la revendication 1, dans lesquels
R¹ représente un atome d'hydrogène; un groupe alkyle contenant de 1 à 10 atomes de carbone, un groupe alcényle contenant de 3 à 6 atomes de carbone, un groupe halogénalkyle contenant de 1 à 6 atomes de carbone et de 1 à 13 atomes d'halogène identiques ou différents, un groupe alcoxyalkyle contenant respectivement de 1 à 6 atomes de carbone dans les fractions alkyle individuelles, un groupe alcoxyalcoxyalkyle contenant respectivement de 1 à 6 atomes de carbone dans les fractions alkyle individuelles, un groupe alkylthioalkyle contenant respectivement de 1 à 6 atomes de carbone dans les fractions alkyle individuelles, chacun de ces groupes étant à chaîne droite ou ramifiée; un groupe cycloalkyle contenant de 3 à 7 atomes de carbone, un groupe hétérocyclyle saturé contenant de 3 à 7 chaînons et 1 ou 2 hétéroatomes identiques ou différents - en particulier un atome d'azote, un atome d'oxygène et/ou un atome de soufre -; ou un groupe arylalkyle ou un groupe aryle contenant respectivement 6 ou 10 atomes de carbone dans la fraction aryle et éventuellement de 1 à 4 atomes de carbone dans la fraction alkyle à chaîne droite ou ramifiée, ou encore un groupe hétéroaryle contenant de 2 à 9 atomes de carbone et de 1 à 3 hétéroatomes identiques ou différents - en particulier un atome d'azote, un atome d'oxygène et/ou un atome de soufre -, chacun de ces groupes portant éventuellement, dans la fraction aryle, respectivement hétéroaryle, de 1 à 5 substituants identiques ou différents, dans lesquels, comme substituants du groupe aryle, respectivement du groupe hétéroaryle, on envisage respectivement:
un atome d'halogène, un groupe nitro; un groupe alkyle, un groupe alcoxy, un groupe alkylthio, un groupe alkylsulfinyle ou un groupe alkylsulfonyle contenant respectivement de 1 à 4 atomes de carbone, chacun de ces groupes étant à chaîne droite ou ramifiée; ainsi qu'un groupe halogénalkyle, un groupe halogénalcoxy, un groupe halogénalkylthio, un groupe halogénalkylsulfinyle ou un groupe halogénalkylsulfonyle contenant respectivement de 1 à 4 atomes de carbone et de 1 à 9 atomes d'halogène identiques ou différents, chacun de ces groupes étant à chaîne droite ou ramifiée, et
R² représente un atome d'hydrogène; un groupe alkyle contenant de 1 à 10 atomes de carbone, un groupe halogénalkyle contenant de 1 à 6 atomes de carbone et de 1 à 13 atomes d'halogène identiques ou différents, ou encore un groupe alcoxyalkyle contenant respectivement de 1 à 6 atomes de carbone dans les fractions alkyle individuelles, chacun de ces groupes étant à chaîne droite ou ramifiée, ou bien
R¹ et R² représentent, ensemble avec l'atome de carbone auquel ils sont liés, un groupe cycloalkyle ou un groupe hétérocyclyle saturé ou insaturé, contenant respectivement de 3 à 8 atomes de carbone et éventuellement 1 ou 2 hétéroatomes identiques ou différents - en particulier un atome d'azote, un atome d'oxygène et/ou un atome de soufre -, chacun de ces groupes portant éventuellement de 1 à 4 substituants identiques ou différents, dans lesquels, comme substituants, on envisage respectivement:
un atome de fluor, un atome de chlore, un atome de brome, un groupe alkyle à chaîne droite ou ramifiée contenant de 1 à 4 atomes de carbone, un groupe alcanoyle à chaîne droite ou ramifiée contenant de 1 à 5 atomes de carbone, un groupe alcanediyle à chaîne droite ou ramifiée contenant de 3 à 6 atomes de carbone, un radical dioxyalkylène à chaîne droite ou ramifiée contenant de 1 à 6 atomes de carbone, un groupe halogénalkyle à chaîne droite ou ramifiée contenant de 1 à 4 atomes de carbone et de 1 à 9 atomes d'halogène identiques ou différents, un groupe alcoxy à chaîne droite ou ramifiée contenant de 1 à 4 atomes de carbone, un groupe alkylthio à chaîne droite ou ramifiée contenant de 1 à 4 atomes de carbone, un groupe cycloalkyle contenant de 3 à 7 atomes de carbone ou un groupe phényle,
R³ représente un atome de fluor, un atome de chlore, un atome de brome, un groupe alkyle à chaîne droite ou ramifiée contenant de 1 à 6 atomes de carbone ou un groupe alcoxy à chaîne droite ou ramifiée contenant de 1 à 6 atomes de carbone,
R⁴ représente un atome d'hydrogène, un atome de fluor, un atome de chlore, un atome de brome, un groupe alkyle à chaîne droite ou ramifiée contenant de 1 à 6 atomes de carbone, un groupe halogénalkyle à chaîne droite ou ramifiée contenant de 1 à 6 atomes de carbone et de 1 à 13 atomes d'halogène identiques ou différents, ou encore un groupe alcoxy à chaîne droite ou ramifiée contenant de 1 à 6 atomes de carbone,
R⁵ représente un atome de fluor, un atome de chlore, un atome de brome, un groupe alkyle à chaîne droite ou ramifiée contenant de 1 à 6 atomes de carbone ou un groupe alcoxy à chaîne droite ou ramifiée contenant de 1 à 6 atomes de carbone, et
n représente le chiffre 0, 1 ou 2.

7. Composés de formule (I) selon la revendication 1, dans lesquels
R¹ représente un atome d'hydrogène; un groupe alkyle contenant de 1 à 8 atomes de carbone, un groupe alcényle contenant de 3 à 5 atomes de carbone, un groupe halogénalkyle contenant de 1 à 4 atomes de carbone et de 1 à 9 atomes d'halogène identiques ou différents, un groupe alcoxyalkyle contenant respectivement de 1 à 3 atomes de carbone dans les fractions alkyle individuelles, un groupe alcoxyalcoxyalkyle contenant respectivement de 1 à 3 atomes de carbone dans les fractions alkyle, un groupe alkylthioalkyle contenant respectivement de 1 à 3 atomes de carbone dans les fractions alkyle individuelles, chacun de ces groupes étant à chaîne droite ou ramifiée, un groupe cycloalkyle contenant de 3 à 6 atomes de carbone, un groupe hétérocyclyle saturé contenant 3, 5 ou 6 chaînons et 1 hétéroatome - en particulier un atome d'azote, un atome d'oxygène ou un atome de soufre -; ou un groupe phénylalkyle ou un groupe phényle contenant éventuellement de 1 à 3 atomes de carbone dans la fraction alkyle, ou encore un groupe hétéroaryle - en particulier un groupe pyridyle, un groupe imidazolyle, un groupe pyrazolyle, un groupe triazolyle ou un groupe thiazolyle -, chacun de ces groupes portant éventuellement, dans la fraction phényle, respectivement hétéroaryle, de 1 à 3 substituants identiques ou différents, dans lesquels, comme substituants du groupe phényle, respectivement du groupe hétéroaryle, on envisage respectivement:
un atome de fluor, un atome de chlore, un atome de brome, un groupe nitro, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe n-, i-, s- ou t-butoxy, un groupe méthylthio, un groupe éthylthio, un groupe méthylsulfinyle, un groupe méthylsulfonyle, un groupe trifluorométhyle, un groupe difluorométhyle, un groupe trifluorométhoxy, un groupe difluorométhoxy, un groupe trifluorométhylthio, un groupe trifluorométhylsulfinyle, un groupe trifluorométhylsulfonyle, et
R² représente un atome d'hydrogène; un groupe alkyle contenant de 1 à 6 atomes de carbone, un groupe halogénalkyle contenant de 1 à 4 atomes de carbone et de 1 à 9 atomes d'halogène identiques ou différents, ou encore un groupe alcoxyalkyle contenant respectivement de 1 à 4 atomes de carbone dans les fractions alkyle individuelles, chacun de ces groupes étant à chaîne droite ou ramifiée, ou bien
R¹ et R² représentent, ensemble avec l'atome de carbone auquel ils sont liés, un groupe cycloalkyle ou un groupe hétérocyclyle saturé ou insaturé, contenant respectivement 3, 5, 6, 7 ou 8 atomes de carbone et éventuellement 1 ou 2 hétéroatomes identiques ou différents - en particulier un atome d'azote, un atome d'oxygène et/ou un atome de soufre -, chacun de ces groupes portant éventuellement de 1 à 3 substituants identiques ou différents, dans lesquels, comme substituants, on envisage respectivement:
un atome de fluor, un atome de chlore, un atome de brome, un groupe alkyle à chaîne droite ou ramifiée contenant de 1 à 4 atomes de carbone, un groupe alcanoyle à chaîne droite ou ramifiée contenant de 1 à 4 atomes de carbone, un radical dioxyalkylène contenant de 1 à 3 atomes de carbone, un groupe halogénalkyle contenant 1 ou 2 atomes de carbone et de 1 à 5 atomes d'halogène identiques ou différents, un groupe alcoxy à chaîne droite ou ramifiée contenant 1 ou 2 atomes de carbone, un groupe alkylthio à chaîne droite ou ramifiée contenant 1 ou 2 atomes de carbone, un groupe cycloalkyle contenant 3, 5 ou 6 atomes de carbone ou un groupe phényle,
R³ représente un atome de fluor, un atome de chlore, un atome de brome, un groupe alkyle à chaîne droite ou ramifiée contenant de 1 à 4 atomes de carbone ou un groupe alcoxy à chaîne droite ou ramifiée contenant de 1 à 3 atomes de carbone,
R⁴ représente un atome d'hydrogène, un atome de fluor, un atome de chlore, un atome de brome, un groupe alkyle à chaîne droite ou ramifiée contenant de 1 à 4 atomes de carbone, un groupe halogénalkyle contenant 1 ou 2 atomes de carbone et de 1 à 5 atomes d'halogène identiques ou différents, ou encore un groupe alcoxy à chaîne droite ou ramifiée contenant de 1 à 3 atomes de carbone,
R⁵ représente un atome de fluor, un atome de chlore, un atome de brome, un groupe alkyle à chaîne droite ou ramifiée contenant de 1 à 4 atomes de carbone ou un groupe alcoxy à chaîne droite ou ramifiée contenant de 1 à 3 atomes de carbone, et
n représente le chiffre 0 ou 1.
